Europäisches Patentamt

**European Patent Office**

Office européen des brevets

(19)

(11) Publication number: **0 433 394 B1**

(12)

# EUROPEAN PATENT SPECIFICATION

(45) Date of publication of patent specification: **08.11.95**

(51) Int. Cl.6: **C05C 9/00**, C05G 5/00, A01C 1/06

(21) Application number: **89911155.3**

(22) Date of filing: **05.09.89**

(86) International application number: **PCT/US89/03771**

(87) International publication number: **WO 90/02719 (22.03.90 90/07)**

(54) **METHOD OF APPLYING ENERGY, CARBON SKELETON AND NUTRIENT MATERIALSTO VEGETATION.**

(30) Priority: **09.09.88 US 242951**
**19.05.89 US 354155**

(43) Date of publication of application:
**26.06.91 Bulletin 91/26**

(45) Publication of the grant of the patent:
**08.11.95 Bulletin 95/45**

(84) Designated Contracting States:
**DE FR GB IT**

(56) References cited:
**EP-A- 0 161 395**
**US-A- 3 640 698**
**US-A- 3 753 722**
**US-A- 4 033 745**

**DATABASE WPI, accession no. 67-04273H [00], Derwent Publications Ltd, London,GB; & JP-A-68 022 206 (SAKAI OTSUKA KAGAKU YAKUHIN CO., LTD)**

(73) Proprietor: **YAMASHITA, Thomas T.**
**2030 North Berkeley Avenue**
**Turlock, CA 95380 (US)**

(72) Inventor: **YAMASHITA, Thomas T.**
**2030 North Berkeley Avenue**
**Turlock, CA 95380 (US)**

(74) Representative: **Baverstock, Michael George Douglas et al**
**BOULT, WADE & TENNANT**
**27 Furnival Street**
**London, EC4A 1PO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

This invention relates to a method of treating plants to stimulate their growth and/or their production of edible or other useful products such as fruits, nuts, etc.

EP-A-0 161 395 describes foliar fertilizers comprising buffer mixtures to control pH on the leaf, which are applied from disposition in water or from the non-aqueous solutions on to the leaf, the adhesiveness of the fertilizers being optionally increased by addition of soluble or insoluble adhesive agents, molasses and sugar being exemplified. In the compositions specifically exemplified the major non-aqueous component is a water-insoluble carbonate or phosphate.

JP-A-68 022 206 describes liquid fertilizer solutions for foliar application containing N, P, K, Mg and Ca, micronutrients such as Mn, B, Fe, Cu, Zn and Mo, urea, an organic acid selected from citric, malic, tartaric and succinic acids, EDTA and optionally a sugar and a surfactant. The solution is diluted 300 to 2000 fold before spraying onto the plant.

Traditional plant nutrition has, to date, approached remedial programs through a chronological path of observation, tissue and/or soil analysis, diagnosis, followed by remedy. Such an approach presupposes and accepts certain natural-occurring phenomena as limitations, the realm in which the plant must necessarily function:

(1) that the plant must operate within and as such is constrained by an array of existing environmental factors such as climate and weather, the atmospheric concentration of carbon dioxide (0.03%), duration and intensity of light, the seasons, limiting edaphic factors, etc.

(2) that the plant must obey certain natural "time" frames of growth and reproduction.

(3) that traditional irrigation, fertilization and pest control strategies will express the full potential of a plant's growth and reproduction.

(4) that the application of some predetermined, deficient nutrient(s) at a specified time and rate will restore the plant to its optimal condition.

(5) that the plant is totally resigned to "autotrophism" and as such must conform to this mode of growth, alone.

An example of a current used technique to enhance growth and/or crop production of plants and of its limitations is as follows: Nitrogen added as a fertilizer or plant nutrient may be in the form of pentavalent (oxidized) nitrogen such as a nitrate or in the trivalent (reduced) form such as ammonia or urea. Assuming that the nitrogen applied to a plant is converted to a protein in which the nitrogen is trivalent, if the form of the nitrogen added is a nitrate it must be converted to the trivalent form which requires a considerable expenditure of energy over and above what is required if the nitrogen is applied in the form ammonia or urea. The energy required must come from tissues of the plant directly or through photosynthesis. This would indicate that the application of nitrogen as ammonia or urea would place less demand upon the plant. However the application of nitrogen wholly as ammonia or urea has or may have disadvantages such as:

(1) a sudden drain of both carbon skeletons and energy.

(2) as a result of the condition created in No. 1, a low carbohydrate:nitrogen ratio promoting vegetative but marginal reproductive growth.

(3) inhibition of photosynthetic electron transport by the ammonium ion.

(4) urea-mediated denaturation of proteins through disruption of sulfhydryl bonds.

Another approach is to add a carbohydrate, such as sugar, directly, for example by a foliar spray of a sucrose or other water soluble, assimilable form of carbohydrate. The sugar, when absorbed into the leaves, will provide a source of energy and also a source of carbon skeleton from which, for example, proteins can be synthesized by the plant. This can be, and often is, a very expensive way in which to apply a source of energy and of carbon skeleton. Also if carbohydrate fractions, alone, are added to the plant, various minerals would be needed to compensate for corresponding demands on balanced physiology. Under greenhouse conditions using daily, complete nutrient fertilizers (such as Hoagland's Solution) and a full range of controlled climatic and other environmental factors, the otherwise sudden physiological imbalances brought on by carbohydrate additions alone could be mollified. Resultingly, this would tend to be manifested in increased growth responses. Under actual field conditions, however, these same isolated additions of beneficial carbohydrates would tend to create offsetting physiological imbalances and would not manifest in full the potential benefits of these treatments.

D. Barel and C.A. Clark in Plant and Soil, Vol. 52, pages 515-525 (1979) disclose a composition for foliar application comprising sucrose together with phosphorous and nitrogen compounds in which the latter are present in amounts smaller than the sucrose.

It is an object of the present invention to provide improvements in the application of nutrients and energy sources to plants especially in consideration of highly variable edaphic and climatic factors, pest and disease pressures and various cultural practices experienced and exercised in both commercial and home-garden farming. Furthermore, presently exercised practices in commercial agriculture, out of economic necessities, place and demand unnaturally productive outputs from the plant. Additionally, all of such vintage productivities are demanded of the plant using traditional, natural cultural practices. It is no wonder then, that farmers are persistently witness to such maladies of the commercial flora as alternate cycles of production, quality variations and shortened productive life, to name a few.

It is a particular object of the invention to provide a method of stimulating the growth of plants and/or the yield of crops or other useful products and to provide compositions which are useful in the practice of such method especially with respect to the aforementioned conditions which beleaguer present day agriculture.

The essential features of the invention are the subject-matter of independant claims 1 and 6, preferred features of the invention are the subject-matter of dependant claims 2-5 and 7-17.

In the preferred CBN composition[1] the following additional component is also present:

Enhancement agent component.

A buffer is also used to adjust the pH of the composition.

Example 1 below illustrates a composition, sometimes referred to as Bright Sun, which is useful in the practice of the invention.

EXAMPLE 1

Sugar beet molasses was used as stock material and source of energy and carbon skeleton. The total invert sugar (TSI) level was brought to 40% by dilution with water. Following are ingredients used to make the molasses blend:

| Macronutrients | | (Elemental) % w/v | Source of Element |
|---|---|---|---|
| Nitrogen | (N) | urea (0.65, KNO3 (0.60) total= 1.25% | Urea, Potassium nitrate |
| Phosphorus | (P) | 1.5 | Phosphoric acid |
| Potassium | (K) | 2.0 | Potassium nitrate |
| Calcium | (Ca) | 2.0 | Calcium gluconate |
| Magnesium | (Mg) | 0.5 | Magnesium sulfate |
| Sulfur | (S) | 3.5 | Various sulfates |

[1] CBN signifies "compensatory balanced nutrition"

### Micronutrients

| | | | |
|---|---|---|---|
| Zinc | (Zn) | 1.0 | Zinc sulfate |
| Iron | (Fe) | 1.0 | Ferrous sulfate |
| Manganese | (Mn) | 1.0 | Manganese sulfate |
| Copper | (Cu) | 0.5 | Cupric sulfate |
| Boron | (B) | 0.02 | Boric acid |
| Molybdenum | (Mo) | 0.03 | Ammonium molybdate |
| Cobalt | (Co) | 0.03 | Cobalt nitrate |

### Vitamins and Cofactors

| | | | |
|---|---|---|---|
| Thiamine | (B1) | 0.02 | Thiamine hydrochloride |
| Riboflavin | (B2) | 0.02 | Riboflavin |
| Nicotinic acid | | 0.02 | Nicotinic acid |
| Pyridoxine | (B6) | 0.02 | Pyridoxine hydrochloride |
| Folic acid | | 0.02 | Folic acid |
| Biotin | | 0.02 | Biotin |
| Pantothenic acid | | 0.02 | Pantothenic acid (calcium salt) |
| Cyanocobalamin | | 0.02 | Vitamin B12 |
| Phosphatidylcholine | | 0.02 | Lecithin |
| Inositol | | 0.02 | Inositol |
| Para-aminobenzoic acid | | 0.02 | PABA |

### Enhancement Agents

| | | |
|---|---|---|
| Seaweed extract | 2.5% (v/v) | Seaweed extract (cold processed) |
| Citric acid | 2.6g/L (10.0gr/gal)mix | Citric acid |
| Katy-J Complexing Agent | 132mg/L (0.5gr/gal)mix | Katy-J (JKT Corp.) |
| Xanthan gum | 0.07 (v/v) | Xanthan gum |

### Sugars and Carbon Skeletons

| | | |
|---|---|---|
| Molasses | 40% (TSI) | Beet molasses |

## Buffers

| Phosphate buffer | 0.02% | Phosphate buffer |
|---|---|---|
| (pH=6) | | |

The most important macronutrients are nitrogen, phosphorus, potassium and calcium but it is preferred that the others also be present. The more important micronutrients are zinc, iron and manganese but it is preferred that the others also be present.

## Mixing Instructions

While under rapid mechanical or hydraulic agitation, water and two thirds of the total molasses volume are mixed. The amount of added water should represent approximately 15% of the molasses volume. Ingredients are then slowly metered into the batch in the following order:

1. Citric acid
2. Katy-J Complexing Agent
3. Phosphoric acid
4. Nitrogen
5. Potassium
6. Micronutrients (separately)
7. Vitamins and cofactors
8. Seaweed extract
9. Xanthan gum

Water is again added to the mix to establish a total invert sugar (TSI) concentration of ~40%. As the TSI of molasses may vary, necessary water volumes may vary accordingly.

As the parent molasses may contain potassium concentrations as much as 2.0-7.0%, it may be necessary to omit potassium nitrate. If potassium nitrate is omitted, the nitrogen may be supplied in total by urea (1.25%). Additionally, inositol levels in molasses may reach levels of 5,800-8,000 ppm, in which case this cofactor may be omitted as well. It is important that the pH of the solution be maintained between 5.0-7.5. This latter requirement may be addressed by analyzing the dilution water sources and adjusting extreme deviations with buffers. Approximately one quart of phosphate buffer per hundred gallons of diluted spray mix (i.e. the "Bright Sun" diluted with water for actual spraying) should meet these needs. If the parent molasses has a pH above 7, the standard addition of citric acid and phosphoric acid will adjust this to a manageable level (most molasses have a pH range of between 5-8).

Storing the material between temperatures of 60-80 degrees F is necessary to prolong the activity of ingredients. Dilutions for actual spray applications should try to achieve a final TSI between 4-10% ("Bright Sun" TSI = 40%).

The many crops to be treated may vary in requirements with respect to species, season and an assortment of environmental factors. It would then be necessary to adjust concentrations of the various ingredients. Workable alternative ranges of these concentrations along with alternative sources are presented:

In the above "Katy J" is the trademark of JKT Corporation for a mixture of polyhydroxy organic acids used as a complexing (chelating) agent. Commenting on the enhancement agents, the seaweed extract supplies plant hormones which contribute to regulation of plant metabolism; the citric acid and Katy J serve as complexing or chelating agents and assist in the transport/ingestion of other ingredients of the Bright Sun composition; and the xanthan gum functions as a thickening agent to solubilize ingredients that would otherwise precipitate or drop out.

The phosphate buffer was potassium phosphate.

Alternative sources of the ingredients are listed below.

## Macronutrients

N-ammonium nitrate, monoammonium phosphate, ammonium phosphate sulfate, ammonium sulfate, ammonium phosphatenitrate, diammonium phosphate, ammoniated single superphosphate, ammoniated triple superphosphate, nitric phosphates, ammonium chloride, aqua ammonia, ammonia-ammonium nitrate solutions, calcium ammonium nitrate, calcium nitrate, calcium cyanamide, sodium nitrate, urea, urea-formaldehyde, urea-ammonium nitrate solution, nitrate of soda potash, potassium nitrate, amino acids, proteins, nucleic acids

P-superphosphate (single, double and/or triple), phosphoric acid, ammonium phosphate, ammonium phosphate sulfate, ammonium phosphate nitrate, diammonium phosphate, ammoniated single superphosphate, ammoniated single superphosphate, ammoniated triple superphosphate, nitric phosphates, potassium pyrophosphates, sodium pyrophosphate, nucleic acid phosphates

K-potassium chloride, potassium sulfate, potassium gluconate, sulfate of potash magnesia, potassium carbonate, potassium acetate, potassium citrate, potassium hydroxide, potassium manganate, potassium phosphate, potassium molybdate, potassium thiosulfate, potassium zinc sulfate

Ca-calcium ammonium nitrate, calcium nitrate, calcium cyanamide, calcium acetate, calcium acetylsalicylate, calcium borate, calcium borogluconate, calcium carbonate, calcium chloride, calcium citrate, calcium ferrous citrate, calcium glycerophosphate, calcium lactate, calcium oxide, calcium pantothenate, calcium proprionate, calcium saccharate, calcium sulfate, calcium tartrate

Mg-magnesium oxide, dolomite, magnesium acetate, magnesium bensoate, magnesium bisulfate, magnesium borate, magnesium chloride, magnesium citrate, magnesium nitrate, magnesium phosphate, magnesium salicylate, magnesium sulfate

S-ammonium sulfate, ammonium phosphate sulfate,

calcium sulfate, potassium sulfate, magnesium sulfate, sulfuric acid, cobalt sulfate, copper sulfate, ferric sulfate, ferrous sulfate, sulfur, cysteine, methionine

Micronutrients

Zn-zinc oxide, zinc acetate, zinc bensoate, zinc chloride, zinc citrate, zinc nitrate, zinc salicylate, ziram

Fe-ferric chloride, ferric citrate, ferric fructose, ferric glycerophosphate, ferric nitrate, ferric oxide (saccharated), ferrous chloride, ferrous citrate ferrous fumarate, ferrous gluconate, ferrous succinate

Mn-manganese acetate, manganese chloride, manganese nitrate, manganese phosphate

Cu-cupric acetate, cupric butyrate, cupric chlorate, cupric chloride, cupric citrate, cupric gluconate, cupric glycinate, cupric nitrate, cupric salicylate, cuprous acetate, cuprous chloride

B-calcium borate, potassium borohydride, borax, boron trioxide, potassium borotartrate, potassium tetraborate, sodium borate, sodium borohydride, sodium tetraborate

Mo-molybdic acid, calcium molybdate, potassium molybdate, sodium molybdate

Co-cobaltic acetate, cobaltous acetate, cobaltous chloride, cobaltous oxalate, cobaltous potassium sulfate, cobaltous sulfate

Vitamins and Cofactors

Thiamine-thiamine pyrophosphate, thiamine monophosphate, thiamine disulfide, thiamine mononitrate, thiamine phosphoric acid ester chloride, thiamine phosphoric acid ester phosphate salt, thiamine 1,5 salt, thiamine triphosphoric acid ester, thiamine triphosphoric acid salt, yeast, yeast extract

Riboflavin-riboflavin acetyl phosphate, flavin adenine dinucleotide, flavin adenine mononucleotide, riboflavin phosphate, yeast, yeast extract

Nicotinic acid-nicotinic acid adenine dinucleotide, nicotinic acid amide, nicotinic acid benzyl ester, nicotinic acid monoethanolamine salt, yeast, yeast extract, nicotinic acid hydrazide, nicotinic acid hydroxamate, nicotinic acid-N-(hydroxymethyl)amide, nicotinic acid methyl ester, nicotinic acid mononucleotide, nicotinic acid nitrile

Pyridoxine-pyridoxal phosphate, yeast, yeast extract

Folic acid-yeast, yeast extract, folinic acid

Biotin-biotin sulfoxide, yeast, yeast extract, biotin 4-amidobenzoic acid, biotin amidocaproate N-hydroxysuccinimide ester, biotin 6-amidoquinoline, biotin hydrazide, biotin methyl ester, d-biotin-N-hydroxysuccinimide ester, biotin-maleimide, d-biotin p-nitrophenyl ester, biotin propranolal, 5-(N-biotinyl)-3 aminoallyl)-uridine 5'-triphosphate, biotinylated uridine 5'-triphosphate, N-e-biotinyl-lysine

Pantothenic acid-yeast, yeast extract, coenzyme A

Cyanocobalamin-yeast, yeast extract

Phosphatidylcholine-soybean oil, eggs, bovine heart, bovine brain, bovine liver, L-a-phosphatidylcholine, B-acetyl-g-O-alkyl, D-a-phosphatidylcholine(PTCn), B-acetyl-g-O-hexadecyl, DL-a-PTCh,B-acetyl-g-O-hexadetyl, L-a-PTCh, B-acetyl-g-O-(octadec-9-cis-enyl), L-a-PTCh, B-arachindonoyl, g-stearoyl, L-a-PTCh, diarachidoyl,L-a-PTCh, dibehenoyl (dibutyroyl, dicaproyl, dicapryloyl, didecanoyl, dielaidoyl, 12 diheptadecanoyl, diheptanoyl), DL-a-PTCh dilauroyl, L-a-PTCh dimyristoyl (dilauroyl, dilinoleoyl, dinonanoyl, dioleoyl, dipehntadeconoyl, dipalmitoyl, distearoyl, diundecanoyl, divaleroyl,B-elaidoyl-a-palmitoyl, B-

EP 0 433 394 B1

linoleoyl-a-palmitoyl) DL-a-PTCh di-O-hexadecyl (dioleoyl, dipalmitoyl, B-O-methyl-g-O-hexadecyl, B-oleoyl-g-O-hexadecyl, B-palmitoyl-g-O-hexadecyl), D-a-PTCh dipalmitoyl, L-a-PTCh, B-O,methyl-g-O-octadecyl, L-a-PTCh, B-(NBD-aminohexanoyl)-g-palmitoyl, L-a-PTCh, B-oleoyl-g-O-palmitoyl (stearoyl), L-a-PTCh, B-palmitoyl-g-oleoyl, L-a-PTCh, B-palmitoyl-a-(pyren 1-yl) hexanoyl, L-a-PTCh, B(pyren-1-yl)-decanoyl-g-palmitoyl, L-a-PTCh, B-(pyren-1-yl)-hexanoyl-g-palmitoyl, L-a-PTCh, B-stearoyl-g-oleoyl

Inositol-inositol monophosphate, inositol macinate, myo-inositol, epi-inositol, myo-inositol 2,2' anhydro-2-c-hydroxymethyl (2-c-methylenemyoinositol oxide), D-myo-inositol 1,4-bisphosphate, DL-myo-inositol 1,2-cyclic monophosphate, myo-inositol dehydrogenase, myo-inositol hexanicotinate, inositol hexaphosphate, myo-inositol hexasulfate, myo-inositol E-monophosphate, D-myo-inositol l-monophosphate, DL-myo-inositol l-monophosphate, D-myo-inositol triphosphate, scyllo-inositol

PABA-m-aminobenzoic acid, O-aminobenzoic acid, p-aminobenzoic acid butyl ester, PABA ethyl ester, 3-ABA ethyl ester

## Enhancement Agents

Seaweed extract-kelp extract, kinetin, kinetin riboside, benzyladenine, zeatin riboside, zeatin, extract of corn cockle isopentenyl adenine, dihydrozeatin, indoleacetic acid, phenylacetic acid, indole ethanol, indoleacetaldehyde, indoleacetonitrile, gibberellins (e.g. GA1, GA2, GA3, GA4, GA7, GA38 etc.)

Citric acid-phosphoric acid, acetic acid, proprionic acid, malic acid, isocitric acid, oxalic acid, malic acid, a-ketoglutaric acid, aspartic acid, succinic acid

Katy-J-EDTA, EDDA, EDDHA, EGTA, HEDIA, CDTA, DTPA, NTA, katy-J + EDTA, humic acids, ulmic acid fractions, fulvic acid fractions, leonardite, hymatomelanic acid, lignosulfonic acid, citric acid, phosphatidyl choline

Xanthan gum-guar gum, gum agar, gum accroides, gum arabic, gum carrageenan, gum damar, gum elemi, gum ghatti, gum guaiac, gum karya, locust bean gum, gum mastic, gum pontianak, gum rosin, gum storax, gum tragacanth

## Carbohydrate and Carbon Skeletons

sugar-mannose, lactose, dextrose, arythrose, fructose, fucose, galactose, glucose, gulose, maltose, polysaccharide, raffinose, ribose, ribulose, rutinose, saccharose, stachyose, trehalose, xylose, xylulose, adonose, amylose, arabinose, fructose phosphate, fucose-p, galactose-p, glucose-p, lactose-p, maltose-p, mannose-p, ribose-p, ribulose-p, xylose-p, xylulose-p, deoxyribose, corn steep liquor, whey, corn sugar, corn syrup, maple syrup, grape sugar, grape syrup, beet sugar, sorghum molasses, cane molasses, calcium lignosulfonate

sugar alcohol-adonitol, galactitol, glucitol, maltitol, mannitol, mannitol-p, ribitol, sorbitol, sorbitol-p, xylitol

organic acids-glucuronic acid, a-ketoglutaric acid, galactonic acid, glucaric acid, gluconic acid, pyruvic acid, polygalacturonic acid, saccharic acid, citric acid, succinic acid, malic acid, oxaloacetic acid, aspartic acid, phosphoglyceric acid, fulvic acid, ulmic acid, humic acid

nucleotides and bases-adenosine, adenosine-p, adenosine-p-glucose, uridine, uridine-p, uridine-p-glucose, thymine, thymine-p, cytosine, cytosine-p, guanosine, guanosine-p, guanosine-p-glucose, guanine, guanine-p, NADPH, NADH, FMN, FADH

## Buffers

phosphate buffer-acetate buffer, AMP buffer, calcium tartrate, glycine buffer, phosphate citrate buffer, tris buffer

Of the macronutrients listed above, the most important are N, P, K and Ca but this component preferably also includes magnesium and sulfer.

Of the micronutrients listed above, the most important are Zn, Fe and Mn, but this component preferably also includes the others in the list.

Following is a general description of the method of the invention following which are Examples 2 to 9.

## General Description of Method of the Invention

The rationale of the method of the present invention may be described as follows:

7

Detailed Description of Method

Implementation of CBM Theory requires the following steps:

1. One needs to calculate the energy units within plant tissues of an hypothetical, superior plant; (e.g., fruits, nuts, supportive tissues). This involves the assigning of a calorie value to carbohydrate (CHO), protein and/or fat constituents; the standard free energy of formation of one gram of CHO or protein is approximately 17.2 kJ (4.1 Kcal) and one gram of fat 38.9 kJ (9.3 Kcal). In many cases the CHO, protein and fat constitutions of several crops can be obtained from published literature. When these are unavailable, standard laboratory analyses will provide the information needed. Support tissues such as shoots are examined empirically and their mass estimated as approximately 60% of the wet weight. These tissues are all assigned to CHO caloric value as they are almost entirely of cellutlitic constitution. Standard procedures for estimating shoot growth is conducted by actual counting of the number of current year shoots on a secondary scaffold. The number of secondary scaffolds are then multiplied by the total number of primary scaffolds. This resultant value is multiplied by the number of shoots originally counted to obtain the total number of new shoots per tree (for smaller plants, the entire plant or a larger fraction can be counted). Ten of the largest sized shoots are removed and their wet weight determined. The average weight is multiplied by the total number of shoots and 60% of this value is used as an estimate of the shoot growth. Shoot growth expressed in grams is then multiplied by 17.2 kJ (4.1 Kcal) to arrive at the energy value of these tissues. Because observations of root growth are difficult, an ideal root:shoot ratio of 0.8 is used to estimate the growth and caloric contribution from the roots (i.e. the energy value of shoot growth is multiplied by 0.8 to obtain the root growth caloric value).

The combined caloric values of reproductive and support tissues now represents the estimated energy units within the hypothetical superior plant.

2. The contribution of the primary macronutrient, nitrogen (N), is estimated from protein constituents (calculated in No. 1 above). To estimate the contribution on N in proteins, the author uses a value of 20%, based upon the N in a typical amino acid, lysine. For example, if almonds are made up of 40% protein, then, one pound of almonds contains 1.3 ounces of N (454 grams of almonds x 0.40 x 0.20 = 36.3 grams = 1.3 ounces). The resultant value is doubled to account for nucleic acids, hormones and related compounds which also contain N. This quantity of N represents an estimate of the minimal annual requirement of N.

3. Quantities of N obtained in No. 2 above are assigned energy of assimilation value. As illustrated in the text, approximately 1043 kJ (249 Kcal) are required to assimilate one gram molecular weight of N. The nature of N sources (primarily nitrate vs ammonia forms) may alter the kilocalories required for assimilation (1043 kJ (249 Kcal) required to assimilate nitrate vs 214 kJ (51 Kcal) for ammonia) of N. However, energy of assimilation values are derived from biochemical reactions leading up to the incorporating of N into one protein. This does not take into consideration alternate paths of transaminations and/or biochemical transformations. Thus, the author elects to utilize the energy of assimilation values in relation to utilizing nitrate as a sole N source as this is a more realistic estimate of actual energies utilized by a plant in assimilating N.

4. The sums of energy requirements calculated in 1 and 3 above, then, represent the theoretical energy demand for the hypothetical superior plant one hopes to achieve.

5. The solar energy harvesting capacity of the untreated plant is estimated. To obtain this, the following are necessary:

a. estimate of leaf surface area in square meters; the number of leaves are counted from a tertiary or quaternary scaffold (small plants may be counted in their entirety) and multiplied by the appropriate factor; the total number of leaves is multiplied by the area of a typical leaf.

b. 5.78 Einsteins of energy will strike a square meter in one hour; this is equivalent to approximately 1047 kJ (250 Kcal)/square meter/hour (note: this considers an average sunny summer day).

c. the author uses a 10 hour day and the number of equivalent sunny summer days during the growing season of the plant.

Total leaf surface x total hours x 181 kJ (43.2 Kcal)/sq. meter/hour are multiplied to obtain the potentially harvestable energy.

6. The kJ (Kcal) value obtained in No. 5 represents the potential harvestable solar energy. However, actual photosynthetic efficiency of plants runs between 0.5%-3.5%. Percentage designation is based on the following table:

| Maximum Photosynthetic Rates of Major Plant Types Under Natural Conditions | | | |
|---|---|---|---|
| Type of Plant | Appr. P.E.* | Example | Max. Phot.** |
| CAM (Crassulacian acid metabolism) | 0.5% | succulents (Agave americana) | 1-4 |
| Tropical, subtropical mediterranean evergreen trees and shrubs; temperate zone evergreen conifers | 1.0% | Scotch Pine (Pinus sylvestris) | 5-15 |
| Temperate zone deciduous trees and shrubs | 1.25% | European beech (Fagus sylvatica) | 5-20 |
| Temperate zone herbs and C-3 pathway crops | 2.0% | soybean (Glycine wax) | 15-30 |
| Tropical grasses, dicots and sedges with C-4 pathways | 3.5% | corn or maize (Zea mays) | 35-70 |

\* Approximate Photosynthetic Efficiency
\*\* Maximum Photosynthesis (mg $CO_2$/dm2/hour) (from: W. Larcher, 1969, Photosynthetica 3:167-198)

Thus, the value from No. 5 is multiplied by the appropriate efficiency to obtain actual harvest solar energy per season.

7. The energy demand (No. 4) is subtracted from the actual harvestable solar energy (No. 6). If the value is negative, this represents a deficit in energy which must be compensated to achieve the hypothetical superior plant.

8. In most cases a deficiency of energy units will have to be compensated with Bright Sun applications. Application programing is based on the following criteria:

   a. early spring growth should be applied as a 4-5% TSI concentration

   b. later growth can be treated with 8-10% TSI solutions

   c. the specific goals of a program will dictate frequency of applications - e.g. if one is trying to overcome alternate bearing in pistachios it is critical that at least 3 applications are applied between early April and mid-May when the shoots bearing next year's fruit buds will be determined; as a general rule, prelog and log phase growth periods are most demanding of energy and nutrients, followed next by the linear and senescence phases (see graph below) (from: W.G. Whaley, 1961, in W. Ruhland, ed., Encyclopedia of Plant Physiology, Volume 14, Springer-Verlag, Berlin, pp. 71-112)

9. Most of the carbon skeleton-energy sources such as sucrose and other Bright sun constituents will have entered the plant tissues within 4 days. The author has observed that under spring and summer conditions most plants will manifest noticeable growth 10-14 days following a Bright Sun application. These new tissues not only represent rapidly metabolizing centers, but their relative succulence in combination with this factor facilitate absorption of Bright Sun. It is known that microscopic passage canals, the ectoteichodes, provide communication channels with the outside environment and thus are avenues for absorption of compounds and elements. With the appropriate use of surfactants it may be possible to get materials through the stomata as well. Further, actively transported compounds, which thus require ATP, may gain additional help by the increased oxygen absorption induced by both "salt respiration" and added metabolizable energy units. Nonetheless, taking advantage of rapidly metabolizing, succulent tissues further enhances material absorption and this factor serves as a sound basis for instituting 10-14 day repeat application schedules. Additionally, by 10-14 days localized depletion of elements and/or energy may begin to appear. It is necessary, then, to compensate for the induced increases in metabolism by periodic applications of Bright Sun until the plant is conditioned (about midpoint or further beyond the linear phase of growth) to operate for the remainder of the season at its induced, higher, efficiency level. The more applications per season, the more benefits to the plant. The following table may serve as an example.

| Effect of number of sprays with 10% sucrose solution on growth of tomato variety San Jose Extra Early | | |
| --- | --- | --- |
| No. Sprays | Mean total dry wt/mg | Dry wt increase |
| 0 | 188 | - |
| 1 | 204 | 16 |
| 2 | 229 | 41 |
| 3 | 238 | 50 |
| 5 | 281 | 93 |
| 10 | 352 | 163 |
| 20 | 596 | 408 |
| Note: duration of experiment 21 days (from: A.M.M. Berrie, Physiologia Plantarum 13, 1960) | | |

Compensation of deficient energy units is only partly met by a direct addition. That is let us assume, for example, that a tree requires 418,680 kJ (100,000 Kcal) to produce 11 kg (25 lbs.) of nuts (dry wt.) but can at most harvest 251,200 kJ (60,000 Kcal) of sunlight during the season. If the biological combustion of one mole of sucrose yields 2202 kJ (526 Kcal), simple division (40,000 divided by 526 = 76 moles of sucrose) indicates a need for about 76 moles of sucrose. At 342 grams per mole, direct compensation of energy, then, would require almost 27 kg (59 lbs.) of sugar. Obviously, it would be far too difficult and expensive to add this quantity directly. However, if repeated applications of Bright Sun (5-10% TSI) were practiced at periodic intervals to gradually increase the overall metabolic efficiency and capacity of the plan, the 27 kg (59 lbs.) of sucrose energy would be added indirectly. The addition of sucrose in foliar sprays, for example, is known to improve the plant in a number of ways:

1. delaying senescence
2. increase the number of plastids per cell (including chloroplasts and mitochondria
3. increase thylakoid formation
4. increase thylakoid polypeptides
5. increase cellulose synthesis
6. increase the rate and amount of organic acids secreted by roots, thus improving the ability to extract mineral elements from the soil
7. increase the rate of differentiation of cells
8. stimulate cyclic AMP formation, thus regulating intracellular metabolism leading to increased enzyme activity and overall metabolic efficiency.

Additionally it is known that the application of metal activators, cofactors and coenzymes will not only institute activity of an enzyme but by virtue of the former effect greatly accelerate the rate and efficiency of biochemical reactions. Growth promoting, plant hormones also act in a regulatory capacity and as such can act in a similar fashion. When a full range of factors (as found in Bright Sun) are then used in applications to a plant, the potential voids in one or a number of related factors created by accelerated activity from additions of another are nullified. This is so because of the complete, balanced nature of the Bright Sun mix which will allow compensation of an otherwise deficient factor or factors.

If, for example, one is able to increase the leaf surface area of the given tree by 40%, theoretically, the tree would be able to harvest an additional 100,000 kJ (24,000 Kcal) (60,000 Kcal x .40 = 24,000 Kcal). If the metabolic efficiency of the same tree is improved by 30%, an additional 75,000 kJ (18,000 Kcal) of harvested energy would be possible. The sum of these (24,000 Kcal + 18,000 Kcal = 42,000 Kcal) or 175,000 kJ (42,000 Kcal) would more than compensate for the deficiency of 167,000 kJ (40,000 Kcal) (60,000 Kcal + 42,000 Kcal = 102,000 Kcal, with a requirement of 100,000 Kcal). It is by virtue of these phenomena that a superior plant is produced by treatments of Bright Sun without having to directly compensate an energy deficiency. Rather, it is the combined effects of a minute direct addition along with the all important improvement in overall metabolic efficiency which makes it possible to achieve the status of a superior plant. It is the inclusion of a carbon skeleton-energy source in conjunction with additions of macro and micronutrients, cofactors and coenzymes, growth regulators, complexing agents and related factors that prevents a temporary energy deficit within the plant. That is, energies of assimilation for various elements and compounds are compensated from the beginning of treatment and are not met at the total expense of the plant's reserve energy sources. Thus, a break in metabolic efficiency is avoided and increased rates of metabolism induced by treatments are allowed to continue unimpeded. Under traditional methods of plant nutrition it is not uncommon to create a deficiency or imbalance in the biochemical machinery following treatments with one or more elements.

Compensatory Balanced Nutrition avoids these imbalances by providing a full range of factors at specific ratios designed to promote both growth and reproduction (or growth alone, as with a specific ornamental, e.g.). However, final application scheduling must correlate the benefits to the plant with economic returns to the grower.

The macroscopic manifestations in plants often translates into characters such as increased growth, bud retention, fruit size and quality as well as subtle expressions of tolerance to various forms of environmental stress. A generalized definition of these beneficial factors, then, must emphasize balance and the concepts of "compensatory balanced nutrition". That is, the addition of one factor, such as nitrogen, must take into consideration concomitant needs for energies of assimilation, carbon skeletons to accept nitrogen, the need for cofactors and catalysts and a wide range of other macro and micronutrients. The enhanced rate and activity of a series of biochemical reactions must necessarily create temporary states of deficiency or excess. A "compensatory balance" approach, however, takes all the myriad of factors into consideration. If we were to assign a relative value to these many factors, however, with all else being normal, it is obvious that the energy load of the plant represents the ultimate factor of limitation.

It is the purpose of this patent to emphasize these concepts and to demonstrate the necessity of integrating a "compensatory balanced nutrition" (CBN) of plants. Traditional plant nutrition has to date only addressed the need for various mineral elements. While results may appear to be favorable the potentials have yet to be realized. Rather, by addressing the additional energy requirements and certain key cofactors (such as vitamins) it is possible to achieve growth and production which exceed even the most balanced nutrition of mineral elements.

Summary of Description of Method

1. Establish an optimum and/or desired crop level (e.g. tons/acre or kg/m$^2$).
2. Select a plant of superior framework capable of supporting the mass and volume of crop necessary to meet the established optimum crop in No. 1 above.
3. Determine the energy and nitrogen-phosphorus-potassium (NPK) + calcium (Ca) + magnesium (Mg) levels necessary to support all growth during the course of a season for both the plant and crop in No. 1 and 2 above (and also for an average, typical plant). This will include:
    a. All vegetative growth put forth during the current season
        1) roots
        2) shoot growth
        3) increases in girth (expansive growth) for past season growth (e.g. as in tree branches)
    b. All crop tissue (e.g. fruits, nuts, seeds, etc.)
    Note:    N, P, K, Ca and Mg levels can usually be obtained from published literature and will be expressed as a percent of dry tissue weight; energy levels are determined from the following:
    c. Carbohydrate (CHO), protein (Prot) and fat constituents making up vegetative and crop growth are determined:
        1) CHO and Prot constituents are assigned a value of 17.2 kJ/gram (4.1 Kcal/gram)
        2) Fat constituents are assigned a value of 38.9 kJ/gram (9.3 Kcal/gram)
    d. Each gram molecular weight of N is assigned 1047 kJ (250 Kcal); P, K, Ca and Mg do not receive Kcal designations
4. Determine the energy harvesting capacity of the superior plant in No. 2.
    a. Measure the total leaf surface area of the plant

b. Establish a photosynthetic efficiency level for the plant (i.e. the ability to harvest incident light energy and to convert it to energy within the plant)

c. Establish the approximate total energy harvested during the course of a single season (from numbers generated in a and b above)

5. Determine whether or not an energy deficit exists by subtracting the projected, total harvestable energy (in 4c) from the total energy required for the optimum growth and crop (3c and 3d) of the superior plant.

Note: If energy required for growth and crop (3c and 3d) exceeds harvestable energy (4c), a deficit in energy exists.

6. Determine the energy harvesting capacity of an average, typical plant.

Note: The format in No. 4 is followed.

7. Determine the degree of energy deficit that exists when comparing energy demands for an optimum crop versus energy harvesting capacity for the average, typical plant in No. 6.

8. The deficit figured in No. 7 represents the immediate in-season energy deficit that must be accounted for to obtain the optimum crop. The deficit figured in 5 represents the energy deficit to be accounted for in succeeding seasons once the superior framework plant is obtained.

9. Determine the predominant form of translocatable carbohydrate in the specific plant as this provides the guideline as to what form of carbon skeleton-energy source will be utilized in Bright Sun[2] for that specific plant.

Note: This can be obtained from either published literature or by use of standard laboratory procedures.

10. Based on the specific carbon skeleton-energy source selected, the species specific Bright Sun formulation is then applied to the plant and the photosynthetic rate (Pr) monitored daily for 14 days (via $CO_2$ analyzer); the average increase in Pr observed then determines the frequency of applications of Bright Sun necessary to achieve the optimum crop; the following example illustrates this procedure -- assume a case as follows:

(1) The plant is only capable of harvesting 50% of the energy necessary to produce an optimum crop.

(2) The season is 140 days long (i.e. leading up to harvest).

(3) The observed average Pr is 300% (i.e. the increase in Pr following each application of Bright Sun).

(4) If Bright Sun were applied every 14 days (a total of ten applications) a theoretical energy harvest would result in 300% of normal.

(5) If a 50% deficit is the beginning condition, the final energy harvest would more than meet the deficit by a factor of 1.5 (i.e. 1.5 times more energy harvested than would be necessary to just meet the needs for the optimum crop).

(6) Theoretically 0.15 of the optimum crop energy demand would be met with each spray (total of ten sprays).

(7) Thus, to just reach an energy harvest factor of 1.0, approximately seven sprays of Bright Sun would suffice (1.0 divided by 0.15).

(8) If the grower wishes to achieve no more and no less than 1.0 of the required energy demand, a recommendation would be made for seven applications of Bright Sun to be applied every 14 days.

The following Examples 2 to 9 will further serve to illustrate the invention and several different modes of applying the invention.

Example 2 - Almond Trees

Three successive foliar sprays on almonds were utilized to help set the young fertilized nutlets. Each spray was spaced approximately 10-14 days apart. The following mixture was used:

[2]Bright Sun is the formulation of Example 1

| Element | Concentration in Molasses Mix |
|---|---|
| Calcium | 1.0% |
| Potassium | 0.6% |
| Zinc | 0.5% |
| Magnesium | 0.3% |
| Nitrogen | 0.7% |
| Phosphorus | 0.3% |
| Manganese | 0.08% |
| Molybdenum | 0.008% |
| Iron | 0.1% |
| Copper | 0.02% |
| Boron | 0.02% |
| Cobalt | 0.02% |
| Thiamine (B1) | 0.01% |
| Riboflavin (B2) | 0.01% |
| Nicotinic Acid | 0.01% |
| Pyridoxine (B6) | 0.01% |
| Folic Acid | 0.01% |
| Biotin | 0.01% |
| Cobalamin (B12) | 0.01% |
| % invert sugars | 40.00% |

The material has assisted in setting the almond crop. The treated blocks have never set a heavier crop in the 17 year history of the ranch. Additionally, as theorized, the use of these molasses sprays in conjunction with materials developed by the author for frost control, contributed towards protecting the almond crop from incurring major damages. While the neighbor blocks sustained total crop losses in excess of 2428-3238 x $10^3$ m$^2$ (600-800 acres), treated blocks suffered, at most, border damages. This protection occurred under 6-7 continuous hours of 25-26 degrees freezing temperatures.

Example 3 - Pistachio Trees

At present there are several problems encumbering the pistachio industry: (1) verticillium wilt, (2) alternate bearing, (3) nonsplit of shells, (4) embryo abortion and blanking, (5) nut rancidity, and (6) shell staining. It is the belief of the author, following extensive literary, field and laboratory research, that these maladies are all closely tied to improper plant nutrition. For one, verticillium wilt is caused by an opportunistic soil-borne pathogen. During the period of intensive maturation and nut filling (July and August) the developing crop draws upon all available food reserves. Subsequently, the root system sacrifices much of its reserves and at this time root tip necrosis can be observed. These sites, then, serve as entry points for the pathogen. It is interesting to note that V. dahliae falls under the category of a "low sugar pathogen". That is, the organism favors tissues with low concentrations of sugar.

Alternate bearing and related nut quality problems are closely tied to improper nutrition. The calculation of energy flow by the author reveals a deficit in carbohydrates as a primary cause for many of these maladies.

In April the author initiated a foliar spray program to span the months of April through early August (a total of 9 sprays). The purpose was as follows:

1. accelerate the metabolism and upgrade the overall efficiency of the physiological machinery;

2. to add essential elements which not only contribute to goal No. 1, but accounts for and meets the increased demands for these elements;

3. to add energy units and carbon skeletons directly;

4. accelerate abortion of defective nuts at an early stage, thereby leaving the available elemental and energy reserves to perfectly formed healthy nuts;

5. by virtue of No. 4, thin the existing crop and distribute the energy pull of developing nuts over a broader surface;

6. to induce immediate and extensive shoot growth which would give rise to the following year's fruit buds (note: shoot growth and bud differentiation must be completed between the short span of two months, April and May; without it the following year's crop is lost); and

7. to mitigate further infections of verticillium wilt by improving the health of the root system (note: not only does a relatively higher sugar concentration in the root tissue alone reduce the chances for fungal infection but the enhanced rate of root growth allows root tips to literally escape infection as well).

Results of this test thus far are as predicted. Shoot and leaf growth is extensive, measuring anywhere from two to five times the growth seen in neighboring untreated blocks. Defective nuts were aborted 10-14 days in advance of untreated blocks. Shoot growth and concomitant differentiated fruit buds appear very healthy (one can detect this latter condition by observing the size and firmness of the buds). In neighboring untreated blocks many of the fruit buds have abscised, whereas this is not the case in treated blocks. The formulations, concentrations and pertinent information covering these treatments are as follows:

| Element | Concentration in Molasses Mix |
|---|---|
| Nitrogen | 1.2% |
| Phosphorus | 1.0% |
| Potassium | 3.6% |
| Calcium | 1.1% |
| Zinc | 0.5% |
| Magnesium | 0.3% |
| Manganese | 0.2% |
| Molybdenum | 0.01% |
| Iron | 0.3% |
| Copper | 0.025% |
| Boron | 0.02% |
| Cobalt | 0.02% |
| Thiamine (B1) | 0.005% |
| Riboflavin (B2) | 0.005% |
| Nicotinic Acid | 0.005% |
| Paraminobenzoic Acid (PABA) | 0.005% |
| Pyridoxine (B6) | 0.005% |
| Folic Acid | 0.005% |
| Inositol | 0.005% |
| Biotin | 0.005% |
| Cobalamin (B12) | 0.005% |
| Katy-J Complexing Agent | (0.5 grams/acre) 0.124 mg/m$^2$ |
| Citric Acid | (10.0 grams/acre) 2.47 mg/m$^2$ |
| % invert sugars | 40.0% |

First two sprays -

37 mℓ/m$^2$ (40 gpa), 3.7 mℓ/m$^2$ (4.0 gpa) molasses mix, 0.89 m/s (2.0 mph) ground speed; eloctrostatic sprayers with 100% delivery from middle three nozzles and 50% delivery from bottom and top nozzles (note: there are five nozzles per half side of sprayer).

Remaining seven sprays -

37 mℓ/m$^2$ (40 gpa), 3.7 mℓ/m$^2$ (8.0 gpa) molasses mix, 0.89 m/s (2.00 mph) ground speed; electrostatic sprayers with 100% delivery from middle three nozzles and 50% delivery from bottom and top nozzles.

Example 4 - Greenhouse Experiment

A greenhouse experiment was established to further test the feasibility of molasses foliar sprays above and in conjunction with the complexing agent, Katy-J. Chile peppers and "Ace" tomato plants of equal size and age were selected (two per treatment) and potted. One set received twice weekly treatments of 1:9 dilution of molasses:water (of the same blend used in pistachio sprays). A second set received the same in combination with 0.26 g/ℓ (one gram per gallon) (of molasses mix) of Katy-J Complexing Agent. The plants were placed on the lower deck of a greenhouse table to provide shading of all test plants. This was done to provide a suboptimal environmental condition which would assist in accelerating the expression of any differences as a result of treatments.

To date the tomato and pepper plants sprayed with molasses alone are about 25% larger and those with the added Katy-J Complexing Agent up to 50% larger than control plants. The author feels that Katy-J is an important ingredient in these applications. The complex array and quantity of compounds not only added to but also found in the parent molasses necessitates a complexing agent of superior capabilities. An acid test is the ability of Katy-J to keep the metal elements in solution in the presence of phosphorus and calcium. The agent allows drying of the foliar spray on the leaf surface in a state which can later be rehydrated with atmospheric moisture, thereby extending the period of effective absorption. Further data and photographs on these greenhouse tests are forthcoming.

Example 5 - Application to Pollen

Preferred Method For Preparing "SUPER SUN POLLEN"

Closed blossoms ore collected mechanically using a standard shaker-catch frame unit. Blossoms are immediately placed through a low rpm shredder, the cutting teeth of which are replaced by two parallel cylinders revolving inward to direct the flow of product. Cylinders are equipped with sheet metal screws (flat tipped with spiral groove on shaft) which extend from the inside of the cylinder wall to the periphery. The axis of the sheet metal screws pass directly through and ore perpendicular to the central axis of the revolving cylinder. It is the gentle flailing by these teeth which dislodge mature anthers from the blossoms. A shaking deck and two levels of catch frames (one, a coarse 4mm mesh (five-mesh) screen, the other a solid tin frame) provide a preliminary separation of anthers from expended blossoms. Anthers are further separated from extraneous materials through a shaking deck with three levels: (1) top, a 2mm mesh (ten-mesh) stainless steel screen (sss) ; (2) middle, 850$\mu$m mesh (20-mesh) sss; and (3) bottom, a solid frame. Stamens and larger pieces are removed by the upper screen. Viable anthers fall through the first screen and are caught on the second level. Nonviable dehisced anthers, dust and finer extraneous materials ore caught on the lower frame. The motion of the deck action carries product forward. The exit port of each level is staggered to deposit the three classes of material into separate containers.

Pure anthers are then dried on racks lined with fine mesh, breathable nylon fabric. A second method for drying, developed by the author involves the use of slowly revolving perforated cylinders. Anthers are placed into a cylindrical 62$\mu$m mesh (225-mesh) nylon sock, which is cut to fit exactly into the inner diameter and length of the cylinder. A gentle stream of chemically filtered air is directed on the revolving cylinder, which along with the gentle tumbling action, facilitate drying. All drying is done in a dehumidified room with temperatures maintained between 18-25 degrees C. An exhaust system, coupled with an air recirculating system keeps a constant, directed mass flow of air through the building. All recirculated air is purified with permanganate filters which removes harmful concentrations of ethylene and aromatics. Drying is completed when pollen reaches 8-10% moisture. This occurs within 24 hours. The pollen and anthers are then placed on the separating table to further refine the product down to pure pollen grains. for most Prunus and Pyrus species, this is accomplished using a 75$\mu$m mesh (200-mesh) stainless steel screen supported by expanded metal. A gentle rubbing dislodges 95-100% of the pollen grains which fall to a catch frame. This pure pollen is either used immediately, placed under short-term storage (0 degrees C), or placed under long-term storage (-85 degrees C). Pollen is placed in double, vacuum, heat-sealed plastic bags before storage.

Before being distributed out to the field, the "mild-mannered" pollen grains are processed as follows to attain the level of "SUPER SUN POLLEN":

| Item | Proportion | Source |
| --- | --- | --- |
| Pollen grains | 1.0 part | respective species |
| Powdered sugar | 10.0 parts | powdered sugar |
| Katy-J Complexing Agent | 0.2 part | Katy-J (JKT Corp.) |
| Calcium gluconate | 1.0 part | calcium gluconate powder |
| Yeast extract | 1.0 part | yeast extract |

Procedures for Mixing "SUPER SUN POLLEN"

One part of freshly-processed (or recently removed from cold storage) pollen is first mixed with Katy-J to coat the individual grains. One part each of calcium gluconate powder and yeast extract are then added and likewise agitated (shaken in a large, heat sealable bag) to coat pollen grains Ten parts of powdered sugar are blended to complete the pollen mix. The finished product should be immediately vacuum, heat sealed (plastic bag) and kept cold at about 0 degrees C until use. "SUPER SUN POLLEN" is either applied to pollen inserts, sprinkled into the hive and/or applied by aircraft.

Alternate Proportions

| Item | Proportion |
|---|---|
| Pollen grains | 1-10 parts |
| Powdered sugar | 1-100 parts |
| Katy-J | 0.000001-10 parts |
| Calcium gluconate | 0.000001-100 parts |
| Yeast extract | 0.000001-100 parts |

Alternate Sources

| | |
|---|---|
| Katy-J: | Katy-J-EDTA mix, lignosulfonates, fulvic acid, ulmic acid, humic acid, hymatomelanic acid, leonardite, citric acid, isocitric acid, EDTA, EDDA, EDDHA, EGTA, HEDTA, CDTA, DTPA, NTA |
| Calcium gluconate: | calcium (ca) acetate, ca carbonate, ca cyclamate, ca glycerophosphate, ca heptagluconate, ca ionophore, ca-magnesium, ca-phosphate, ca-succinate, ca-tartrate, ca sulfate |
| Yeast extract: | thiamine, riboflavin, nicotinic acid, pyridoxine, folic acid, biotin, pantothenic acid, cyanocobalamin, phosphatidylcholine, PABA (see vitamin and cofactor section for previous "Bright Sun" mix) |

Test Results

1.5% water agar (wa) petri plates (five each) were made up as follows:
A - 1.5% wa
B - 1.5% wa + 10% sugar
C - 1.5% wa + 0.5% calcium gluconate
D - 1.5% wa + 0.5% yeast extract (cold filtered)
E - 1.5% wa + 10% sug + 0.5% ca gluc + 0.5% y ext

Freshly processed pollen grains were lightly sprinkled atop each plate and incubated in the dark for 24 hours. Pollen tube growth was recorded, assigning the A treatment (1.5% water agar, alone) a value of 1 and all others a relative numerical value thereto.

| Replication | | | | | |
|---|---|---|---|---|---|
| Treatment | 1 | 2 | 3 | 4 | 5 |
| A | 1 | 1 | 1 | 1 | 1 |
| B | 19 | 21 | 18 | 21 | 21 |
| C | 7 | 6 | 7 | 5 | 8 |
| D | 5 | 6 | 5 | 8 | 5 |
| E | 23 | 25 | 23 | 24 | 22 |

All blocks on which "SUPER SUN POLLEN" was used show an average crop estimate of 247 g/m$^2$ (2,200 lbs./acre) or better. In some blocks this estimate is close to 336 g/m$^2$ (3,000 lbs./acre) or better. Untreated blocks show average crop estimates all below 224 g/m$^2$ (2,000 lbs./acre). It is also very pertinent that the treated blocks are carrying the heaviest crop in all the 17 year history of the orchards.

Example 6 - Pest Disruptant

Navel Orangeworm Disruptant, "ASUNDER"

Many insect species are directed to hosts and specific host tissues via olfactory stimuli. The mated female navel orangeworm (Amyelois transitella Walker), for example, is attracted to previous year's nuts hanging in the tree (in the spring months) and to the mature nuts. The previous year's nuts or "mummies" which are infested with navel orangeworm(s) (NOW) or other insect larval species (e.g. peach twig borer) are especially attractive to mated females for egg deposition sites.

It has been found that certain fatty acid fractions or crop oils are the key agents of attraction. Foremost among these are the unsaturated fatty acids, linolenic, linoleic and oleic, the latter being most attractive of the three. Crude, unrefined nut and vegetable oils and the acidulated forms of these oils are excellent sources of oleic acid.

During the periods of NOW flight, it may be possible to disrupt the host finding ability of mated females. This is accomplished by permeating the crop environment with attractants that make it virtually impossible for the female to home in on the host tissue(s). Theoretically, it would be possible to halt a generation and avoid extensive damages to the crop.

In March, 1988, the author conducted several studies on the attractability of various candidate compounds. A two-fold goal was to locate a potent attractant oil and a compound that could be used as a nutrient (with comparable attraction and/or which would not nullify the effects of the oil). Black sticky traps were baited with various compounds and placed into orchards having previous histories of heavy NOW infestations. The number of eggs and moths were recorded for two weeks.

| Bait | Eggs |
|---|---|
| NOW bait (bran meal) | 23 |
| Soybean meal | 34 |
| 10% Bright Sun | 34 |
| 5% Crude corn oil | 61 |
| Infested mummy nuts (almonds) | 7 |
| 10% Bright Sun + 5% Crude corn oil | 58 |

Wind tunnel studies: 200 mated females were released downwind from the bait in a confined area, 9mx3mx2.5m (30'x10'x8'); each compound was tested separately for 48 hours each.

| Bait | Females |
|---|---|
| 5% Crude corn oil | 47 |
| Bright Sun | 23 |
| Infested mummy nuts (almonds) | 21 |

Preferred Method for Making "ASUNDER"

| Item | Concentration | Source |
|---|---|---|
| Bright Sun | 55% v/v | Bright Sun |
| Crude almond oil | 40% v/v | Crude almond oil |
| Emulsifier | 5% v/v | Triton X-363M |

Alternate Concentration

| Item | Concentration |
|---|---|
| Bright Sun | 0.000001-75% v/v |
| Crude almond oil | 0.000001-75% v/v |
| Emulsifier | 0.000001-20% v/v |

Alternate Sources

Bright Sun: see alternative mixes in methods for preparing Bright Sun.

Crude almond oil: crude (cd) corn oil, cd cottonseed oil, cd pecan o8il, cd sunflower oil, cd walnut oil, cd filbert oil, cd safflower oil, cd olive oil, cd peanut oil, acidulated (ad) almond oil, ad peanut oil, ad olive oil, ad safflower oil, ad cottonseed oil, ad pecan oil, ad sunflower oil, ad walnut oil, ad peanut oil, oleic acid, linolenic acid, linoleic acid stearic acid, palmitic acid, myristic acid, oleic acid, lauric acid.

Triton X-353M: Bos, Wettal, Pluronic, Plurafac, Iconol, Klearfac, Pluraflo, Armix, Armul, Flomo, Alipal, Blancol, Emulphogene, Emulphor, Gafac, Igepal, Daxad, Agrimul, Hyonic, Monolan, Nopalcol, Atlox, Atphos, Atplus, Atsurf, Brij, Myrj, Renex, Span, Tween, Compex, Pestilizer, Toximul, Surfonic, T-Det, T-Mulz, unimuls, Upanals, Sponto, Atplus 300 F, Lecithin.

Field Test

In May, 1988, the author conducted a large scale field test. Aerial applications were split with one half of the scheduled volume per 4037m$^2$ (per acre) applied on alternate passes. Two weeks later the remainder of the materials were applied to the rest of the fields. During this period and through three weeks into June, 1988, NOW females and eggs were trapped at random locations throughout the treated and untreated blocks. There were 17 traps altogether (both egg traps and moth sticky traps). The results are summarized in the following table:

| Treatment | Maximum No. Eggs for one week | Maximum No. Moths for one week |
|---|---|---|
| Control | 134 | 16 |
| "ASUNDER" | 23 | 1 |
| Note: Control figures represent the readings from five traps; ASUNDER readings were taken from 12 traps; all trapping figures represent the maximum catches for one week over a duration of seven weeks. | | |

Example 7 - Frost Damage Inhibition

Frost Control: "SUNBURST"

Frost concerns represent one of the limiting factors in plant agriculture. Many liquids, including water, can be supercooled below the melting point of the solid phase. Freezing occurs thereafter either spontaneously or in the presence of a catalyst. The catalysts are often referred to as ice nuclei, the two general types of which are classified homogeneous and heterogeneous. Homogeneous nuclei are important below -10 degrees C, while heterogeneous nuclei come into play above this range. Of primary importance to agriculture are freezing temperature ranges between -5 to 0 degrees C. It is at these temperatures that many plant tissues are damaged. That is, supercooling does not occur due to the presence of nuclei catalyzing the liquid to solid transition.

Within this initial freezing range of -5 and 0 degrees C, it has been found that three primary epiphytic bacterial species serve as ice nucleation catalysts (Pseudomonas syringae, Ps. fluorescens, Erwinia herbicola). The bacteria are normal inhabitants colonizing the plant surfaces. It is believed that certain constituents located on the cellular membrane initiate ice formation bringing about freezing and plant tissue damages. Resultingly, programs directed at reducing the populations of ice nucleation bacteria have provided a significant degree of frost protection. Three general avenues of achieving these goals are via the use of:

    1. bactericides

    2. ice nucleation inhibitors

    3. antagonistic bacteria

These approaches relate to findings of a log-linear relationship between frost injury to plants (at a specified temperature) and the quantity of ice nuclei associated with the plant. The lower the population of ice nucleation bacteria, then, the more opportunity for supercooling in the absence of ice formation.

Of the three methods, the use of antagonistic bacteria offers a highly viable and economical means of achieving frost protection. It exercises the principles of microbial ecology of the phylloplane. The soil environment has multiple niches and buffer zones, which contribute to ecological diversity. However, the phylloplane has fewer dimensions and resultingly its extent of diversity is more with respect to time or seasons. An epiphytic bacterial species which aggressively colonises surface tissue, then, encounters few natural obstacles other than variations of moisture and temperature. Thus, once started, a particular colony can be difficult to displace. A logical approach, then, would be to introduce large populations of antagonists following:

    1. previous natural decline of ice nucleating species

    2. bactericidal applications to reduce ice nucleating species

To date investigators have overlooked two key factors for successful introduction of an antagonistic bacterial species:

    1. conditioning the antagonist

    2. providing a temporary substrate on plant surfaces for expansion and an interim for adaptation. The methods developed by the author address these issues.

## Preferred Method for Preparing "SUNBURST"

The previously outlined preferred "Bright Sun" is diluted in the mixing tanks and/or spray rig tank to which is added fermentation and/or plate cultures of naturally occurring antagonists isolated from plant surfaces. The bacteria are not genetically altered but were isolated by the author from almond bud wood. It is a naturally occurring, commonly found species which lives epiphytically on various species of plants. The population is diluted to a concentration of about one billion colony forming units (cfu) per milliliter of dilute spray mix.

Conditioning of the organisms is accomplished by including 0.4% v/v of Bright Sun into the fermentation tank substrate (8 gr/L nutrient broth) or petri plate medium (23 gr/L NA). Subsequently, the spraying of Bright Sun not only serves as a carrier but coats plant surfaces with a temporary substrate for initial colonization. Bright Sun also provides the growing plant with substantial quantities of various nutrients. With an optimal growth status the plant is capable of exuding more of the bacterial growth promoting organic acids and related nutrients.

The following organisms can be used:

    1. Pseudomonas fluorescens (antagonistic strain T-1)

    2. Pseudomonas putida (antagonistic strain K-1)

## Results of Field Tests

| 2-88 | |
|---|---|
| Bright Sun | $3.7\ell/m^2$ (4 gpa) |
| Ps. fluorescens T-1 | ~1 billion cfu per ml |
| Phosphate buffer | $0.25\ell$ per $100\ell$ (1 qt. per 100 gallons) mix |
| Spray volume | $74.8\ell/m^2$ (80 gpa) |

| 3-88 | |
|---|---|
| Bright Sun | 3.7ℓ/m² (4 gpa) |
| Phosphate buffer | 0.25ℓ per 100ℓ (1 qt. per 100 gallons) mix |
| Spray volume | 74.8ℓ/m² (80 gpa) |
| Bright Sun | 3.7ℓ/m² (4 gpa) |
| Ps. fluorescens | T-1 ~1 billion cfu per ml |
| Phosphate buffer | 0.25ℓ per 100ℓ (1 qt. per 100 gallons) mix |
| Spray volume | 74.8ℓ/m² (80 gpa) |

Readings following two consecutive days at approximately 3.3 degrees C (six hours each morning):

| Untreated damaged | 43 of 60 randomly selected nuts |
|---|---|
| "SUNBURST" damaged | 7 of 60 randomly selected nuts |

The frost struck in late May, 1988. Areas which were subjected to similar periods of -3 to -4 degrees C freezing temperatures and which normally sustain yearly damages exceeding 50% losses are holding crops in excess of 224 g/m² (2,000 lbs. per acre). Neighboring untreated plots which were subjected to these temperatures sustained more than 80% frost damage.

Example 8 - Soil Amendment

Soil Amendment: "MORNING SUN"

Continued productivity of our unparalleled agricultural lands faces grave challenges. The basic natural resource and foundation of agriculture, the soil on which we raise our crops, is undergoing chemical and/or physical deterioration over the many years of cropping. Secondly, world expansion and urbanization into otherwise productive locations necessitates development of many virgin lands. However, through the natural processes of geological weathering, these unchartered soils are virtually sterile and unfit for growing profitable crops.

Foremost among soil maladies are salt accumulations and build ups of toxic elements. Furthermore, economical, large scale farming has necessitated the extensive use of herbicide stripping on the effective radius of root growth. Without periodic replenishment of organic matter to these areas, various problems of soil compaction, water penetration and mineral tie up intensify over time. Almost all irrigated regions do not have proper central drainage facilities nor drainage water desalting and recycling plants. Consequently, growers will often take drainage run off, mix this with their irrigation water sources recycling and accumulating salts on their cropland. Aside from establishing efficient drainage and drainage water treatment plants, many growers may improve the quality of the reclaimed marginal croplands with proper soil management.

A central theme of any effective soil management program relies on maintaining the organic matter and thus microbial fractions of the soil. Several species of microbes can harvest atmospheric nitrogen, for example. Under ideal conditions, an entire ecologically coordinated, yet diverse, group of microbes can improve the soil in a myriad of ways:

1. improve structure through formation of aggregated particles
2. increase water retention and availability to roots
3. increase the overall and rate of water drainage
4. improve soil aeration
5. increase the availability of otherwise soil-bound macro and micronutrients
6. add nitrogen to the soil
7. increase the rate of conversion of ammonia forms to nitrates
8. reduce electrical conductivity
9. increase the ion exchange capacity of the soil
10. buffer the plant roots from harmful and toxic levels of chemicals and/or elements
11. degrade harmful chemicals
12. reduce populations of soil-borne plant pathogens and/or reduce the opportunity for their pathogenesis.

The following soil amendment mix addresses these needs:

Preferred Method for "MORNING SUN"

| Item | Concentration | Source |
|---|---|---|
| Part I Mix: | | |
| Bright Sun<br>Katy-J Complexing Agent | parent mix<br>1.3 g/ℓ (5 gr/gal) | Bright Sun<br>mix Katy-J (JKT Corp.) |
| Part II Mix: | | |
| Gloeocapsa sp. | 1 trillion cfu per 3.8ℓ (gallon) mix | fermentation cultures of |
| Streptomyces griseus | " | " |
| Gleocladium roseum | " | " |
| Bacillus subtilis | " | " |
| Pseudomonas fluorescens | " | " |
| Cellulase | 2,500 units/3.8ℓ (gal) | Type VII from Penicillium funiculosum |
| Alpha amylase | 36,000 units/3.8ℓ (gal) | Type XA from Aspergillus oryzae |
| Glycerol | 1.9ℓ (2 qt.)/3.8ℓ (gal) 227g. (8 | glycerol |
| Buffer | oz.)/3.8ℓ (gal) | phosphate buffer |
| Zinc sulfate | 0.05% w/v | zinc sulfate |
| Manganese sulfate | 0.05% w/v | manganese sulfate |
| Iron sulfate | 0.05% w/v | Ferrous sulfate |

The alga species, Gloeocapsa, is cultured in one-half strength Hoagland's Solution supplemented with one gram per 379ℓ (100 gallons) mix of Katy-J. The culture suspension is aerated and provided with constant lighting (via submersible incandescent lamps with an output of light equivalent to approximately 2.0 Einsteins of light energy per square meter per hour). Approximate duration of incubation is 5-7 days. All culturing is conducted under aseptic conditions.

Gleocladium roseum, B. subtilis, S. griseus and Ps. fluorescens are cultured in fermentation tanks similar to that for Gloeocapsa but without lighting and with a different substrate. Nutrient broth (8 gr/L) is supplemented with Bright Sun (0.4% v/v). Pseudomonas fluorescens is a fast grower and is generally mature within 48 hours culturing time. The remaining three species require a minimum culturing period of 72 hours and in many cases 120 hours. All operations are conducted aseptically, under constant, low aeration and at 25 degrees C.

When mature, the cultures are aliquanted and blended with glycerol, phosphate buffer and enzymes. They are placed in breathe-cap containers and refrigerated immediately (5 degrees C). Application involves delivery through the irrigation system or comparable means of approximately one gallon Part I Mix + 1 quart part II Mix per acre (rate may vary with soil condition).

Alternative Concentrations

Part I Mix:

| Bright Sun | see original text on Bright Sun |
|---|---|
| Katy-J | 0.000001-20 (gr/gal) g/3.8ℓ |

Part II Mix:

| Gloeocapsa sp. | 1.0-10(20th) (cfu/gal) cfu/3.8ℓ |
|---|---|
| S. griseus | " |
| B. subtilis | " |
| Ps. fluorescens | " |
| G. roseum | " |
| Cellulase | 1.0-10,000 (units/gal) units/3.8ℓ |
| a-amylase | 1.0-75,000 (units/gal) units/3.8ℓ |
| glycerol | 1.0-90% v/v |
| Buffer | 1.0-10% v/v |
| Zinc sulfate | 1.0-20% w/v |
| Manganese sulfate | 1.0-20% w/v |
| Iron sulfate | 1.0-20% w/v |

Alternate Sources

Part I Mix: (see original text on Bright Sun)

Part II Mix:

| Gloeocapsa sp.: | Anabaena sp. |
|---|---|
| S. griseus: | S. aureofaciens |
| B. subtilis: | B. megaterium, B. cereus, B. brevis |
| Ps. fluorescens: | Ps. putida |
| G. roseum: | Tallaromyces flavus, Trichoderma viride, T. harzianum, Penicillium, citrium, Acremonium falciforme, Ulocladium tuberculatum |
| Cellulase: | Type I (Aspergillus niger), |
| | Type II (A. niger), |
| | Type V (T. viride), |
| | Type VI (T. viride), from T. |

fusca

| a-amylase: | Type IA (porcine pancreas), |
|---|---|
| | Type IIA (Bacillus sp.) |
| | Type XI-A (Bacillus sp.) |
| | Type VI-A, |
| | Type VII-A (porcine pancreas), |
| | Type VIII-A (barley malt) |
| Glycerol: | glycerol |
| Buffer: | see original text on Bright Sun |
| Zn, Mn and Fe sulfates: | see original text on Bright Sun. |

Field Test

Application of 1.123mℓ/m$^2$ (one gallon per acre) of "MORNING SUN" mixes were made on 647,520m$^2$ (160 acres) of pistachio trees, heavily infested with microsclerotia of Verticillium dahliae (150 cfu/gram soil). Eight inch soil cores (25.4mm (1") diameter) were removed from the drip line of five randomly selected trees before and after (2 months) treatment. The soil was air dried, pulverized and the five replicates blended. A 10 gram aliquant was then suspended in 100 ml sterile water. A 1 ml aliquant was removed and plated on 1.5% water agar, allowed to air dry overnight, sealed with parafilm, then placed under two weeks of dark incubation (25 degrees C). Colony forming units (cfu) were read following the two week incubation.

22

| Replicate | cfu before treatment | cfu before treatment |
|-----------|----------------------|----------------------|
| 1 | 6 | 3 |
| 2 | 2 | 1 |
| 3 | 4 | 4 |
| 4 | $\dfrac{3}{15}$ cfu | $\dfrac{1}{15}$ cfu |
| % reduction = 40% | | |

Example 9 - Seed Coating and Root Dip

The soil environment presents a complex range of integrated factors promoting and/or inhibiting plant growth and reproduction. Foremost among the many influential factors is the nature and density of the microbial populations. From the very moment of sowing or planting, the seed or plant roots become enveloped in the dynamic flux of various soil-borne organisms and directly and/or indirectly are affected in subsequent growth. Cultural practices, nature, the basal soil chemistry and microbial populations interact to either favor or impede growth. Various soil-borne pathogens, for example, are opportunistic, gaining entry and/or establishment during weakened states of plant development. Generally speaking, then, adjustments of the soil environment favoring rapid growth and suppressing soil-borne pathogen colonization would subsequently provide opportunity for optimum seed germination, stand, growth and reproduction of the commercial crop.

In recent years a growing awareness of soil ecology has prompted investigations into the science of soil amendments directed towards these ends. Goals have been achieved through modifications of various edaphic factors which would favor growth of existing beneficial populations, by the direct addition of beneficial organisms and a combined effort of both. Supplementary introductions of beneficials have targeted both edaphic enhancing forms as well as antagonists of plant pathogens. The additions of soil amendments has resulted in rather consistent benefits but in large-scale practice has proven to be somewhat cost-limiting. Conversely, the supplementing of antagonists and other beneficials has been met with inconsistencies in results.

The author has explored the nature of these observed phenomena in an attempt to explain inconsistencies and to design cost effective solutions. Invariably, investigators exploring the introduction of beneficials have overlooked the need for concomitant additions of agents which would enhance, their establishment. Secondly, those who have taken the approach of adding soil amendments have done so with primary regard to introducing the end product of ideal microbial activity. Such an approach necessitates massive additions and/or displacement of existing soil. Rather, the author has approached soil improvement whereby minor improvements in certain key edaphic parameters in combination with the introduction of ecologically interrelated populations would achieve near ideal growing conditions. The concept rests heavily upon anticipating a gradual reconstruction of the soil by virtue of timely sequential increases in specific microbial populations. For example, species which can harvest and assimilate nitrogen gas would be a first priority for enhancement. As these populations increase and die off they would provide a substrate for following species. These would add mass and beneficial by-products of their growth such as mucilage, which assists in soil aggregation and thus water penetration, aeration and the release of otherwise bound elements.

Practical avenues for instituting these concepts center about:
1. the addition of bulk volumes of organic matter
2. irrigation drenches with microbial suspensions
3. irrigation introductions of chemicals and/or elements enhancing the chemical and/or microbial environment
4. the coating of seeds and/or roots prior to or during planting.

The author will integrate the above approaches and attempt to exercise their combined virtues via production of the superior seed coating and/or root dip treatment, "SUN COAT".

Preferred Method for Producing "SUN COAT"

| Material | Ratio or Concentration | Source |
|---|---|---|
| Bright Sun<br>Algin<br>Bentonite Clay | 10% v/v<br>2% w/v<br>4% w/v | Bright Sun<br>Keltone LV<br>Bentonite Clay |
| Buffer | 25 mM | 25 mM K2HPO4<br>25 mM KH2PO4 |
| Katy-J<br>Bacillus subtilis<br>Pseudomonas fluorescens<br>Bacillus thuringiensis<br>Gliocladium virens | 530 mg/ℓ (2 gr/gal) mix<br>4x10(12th) (cfu/gal) cfu/3.8ℓ<br>4x10(12th) (cfu/gal) cfu/3.8ℓ<br>4x10(12th) (cfu/gal) cfu/3.8ℓ<br>4x10(12th) (cfu/gal) cfu/3.8ℓ | Katy-J Complexing Agent<br>plate/fermentation cultures<br>plate/fermentation cultures<br>plate/fermentation cultures<br>seed/plate cultures |

Gliocladium virens is first cultured on boiled wheat seeds using the following procedure:

| wheat seeds | 1 cup |
|---|---|
| Bright Sun | 28g (2 oz.) |
| water | 397g (14 oz.) |

The seeds are foiled for approximately 40 minutes then transferred to sterile trays. After cooling, a spore suspension (ca. 1 x 10-6th/ml) is sprayed onto the wheat seeds. Trays are protected with a transparent cover which allows air exchange and incubated at 26 degrees C + low light intensity for approximately 10-14 days. Incolum is collected by placing the spore-covered wheat seeds into a strainer. While agitating, a gentle stream of water is run over the seeds to dislodge spores. The collected spore suspension is then added to the Sun Coat mix.

Bacillus subtilis, B. Thuringiensis and Pseudomonas fluorescens are cultured in fermentation tanks with the following media:

| Nutrient Broth | 10 grams/L |
|---|---|
| Yeast Extract | 10 grams/L |
| Bright Sun | 20 ml/L |
| Phosphate Buffer | 20 mM |
| Water | 1 L |

The ingredients are brought to a boil then autoclaved in flasks at 103 KN/m$^2$ (15 psi), 121 degrees C for 25 minutes. Large-scale operations may replace autoclaving with the use of ultraviolet (UV) lamp sterilizers. The media is first boiled in a concentration about twenty times that of actual usage. It is then diluted with water to the appropriate levels before being pumped through the UV sterilizing unit. The sterilized media is transferred from the UV sterilizer to fermentation tanks equipped with sterile aeration units. Starter cultures of the organisms are grown in shake culture flasks 48 hours prior to their inoculation into fermentation tanks. All cultures are kept at 26 degrees C and under low light intensity. Pseudomonas fluorescens requires 24-49 hours culturing, while B. subtilis and B. thuringiensis may require 72-120 hours.

The parent Bright Sun is then diluted with the suspensions of G. virens, B. subtilis, B. thuringiensis and Ps. fluorescens. Additional water is added to obtain a 10% v/v Bright Sun mixture. To the final diluted mix the following are added:

| Katy-J | (2 grams/gal) 530mg/ℓ |
|---|---|
| Buffer | (16.7 grams K2HPO4/gal) 4.4g/ℓ<br>(13.2 grams KH2PO4/gal) 3.5g/ℓ |
| Bentonite Clay<br>Algin | 4% w/v<br>2% w/v |

During the mixing, it is important to dilute the Bright Sun as far as possible before adding the culture suspensions so as to avoid osmotic stresses on the organisms. Bentonite clay and algin must be added gradually and mixed under high shear agitation to avoid clumping.

The seed to be coated should be soaked and disinfected through a 10% bleach solution for approximately two minutes then immediately and thoroughly rinsed free of bleach with water (this step may be optional depending upon the nature of natural infecting flora). Disinfected seeds are then dipped into the Sun Coat mix, allowed to drain, and placed upon drying trays lined with breathable fabric. A gentle stream of air (not exceeding 35 degrees C) is directed on the seeds to expedite their drying. After about 30 minutes the seeds are placed into a tumbler which individualizes any clumping which had occurred during seed drying.

Alternate Concentrations

| Material | Ratio or Concentration |
|---|---|
| Bright Sun | 1.0-50% |
| Algin | 0.1-10% |
| Bentonite Clay | 0.1-15% |
| Buffer | 0.001-1 M |
| Katy-J | 0.1-50 (grams/gal) g/3.8ℓ |
| Bacillus subtilis | 10 - 1 x 10-25th (cfu/gal) cfu/3.8ℓ |
| B. thuringiensis | 10 - 1 x 10-25th (cfu/gal) cfu/3.8ℓ |
| Pseudomonas fluorescens | 10 - 1 x 10-25th (cfu/gal) cfu/3.8ℓ |
| Gliocladium virens | 10 - 1 x 10-25th (cfu/gal) cfu/3.8ℓ |

Alternate Materials

| | |
|---|---|
| Bright Sun - | see text under Bright Sun |
| Algin - | Xanthan gum, guar gum, gum agar, gum accroides, carboxymethyl cellulose, methyl cellulose, starch, Pelgel, Methocel, gum arabic, gum carragaenan, gum damar, gum elemi, gum ghatti, gum guaiac, gum karya, locust bean gum, gum mastic, gum pontianak, gum rosin, gum storax, gum tragacanth |
| Bentonite Clay - | montmorillonite clay, kaolinite clay, illite clay, amorphous clay, sesquioxide clay, chlorite clay, vermiculite clay, peat, talc, nu-Film 17 |
| Buffer - | succinic acid, malonic acid, hydroxylamine, histidine, cacodylic acid, EDTA (versene), B,B'-dimethylglutaric acid, maleic acid, carbonic acid, citric acid, 4 or 5-hydroxymethylimidazole, pyrophosphoric acid, phosphoric acid, imidazole, 2-aminopurine, ethylenediamine, 2,4,6 collidine, 4 or 5-methylimidazole, triethenolamine, diethylbarbituric acid, tris-(hydroxymethyl) amino-methane, glycyglycine, 2,4 or 2,5-dimethylimidazole, acetate buffer, calcium tartrate, phosphate citrate |
| Katy-J - | see alternatives to Katy-J under Bright Sun |
| Bacillus subtilis - | B. cereus, B. pumilus, B. mycoides, B. megaterium, Thiobacillus ferrooxidans, Actinoplanes missouriensis, A. utahensis, Micromonospora spp., Amorphosporangium auranticolor, Streptomyces griseus, S. aureofaciens, Clostridium butyricum, Glomus mosseae, Bacillus thuringiensis - as above |

| Pseudomonas fluorenscens - | Ps. putida, Enterobacter cloacae, Alcaligines spp., Erwinia herbicola, Agrobacterium radiobacter, Rhizobium japonicum, R. leguminosarum, Serratia liquefaciens, Arthrobacter citreus, A. crystallopoietes, A. globiformis, Pasteuria penetrans, Azotobacter chroococcum, A. paspali, Klebsiella pneumoniae, Nitrosomonas spp., Nitrobacter spp. |
| Gliocladium virens - | G. roseum, Chaetomium globosum, Penicillium oxalicum, P. funiculosum, P. urticae, P. vermiculatum, Trichoderma harzianum, T. hamatum, T. Viride, T. koningii, Fusarium moniliforme variety subglutinans, Pythium nunn, P. oligandrum, Laetisaria arvalis, Coniothyrium minitans, Arthrobotrys amerospora, A. conoides, Acremonium boreale, A. falciforme, Typhula phacorrhiza, Hyphochytrium catenoides, binucleatae Rhizoctonia solani, Talaromyces flavus, Sporodesmium sclerotivorum, Dactylella oviparasitica, Verticillium lacanii, Azolla spp., Gloeocapsa spp., Beauveria bassiana, Ulocladium terculatum |

The specific nature of soil-borne diseases and/or edaphic factors encountered with a particular crop and geographical setting necessitates appropriate adjustments in the organisms and/or ingredients selected in Sun Coat. For this reason, the strains and recipe listed under "preferred methods" represent as close as possible an ideal "general" Sun Coat.

Secondly, seed coating is but one aspect of Sun Coat. The product can also be modified and used as a root dip and/or included with the planting water. A suggested form for the latter two cases is as a powdered product. Excluding the organisms, Bright Sun, algin, bentonite clay, buffer and Katy-J are blended with a minimal volume of water then spray dried. The organisms are cultured and spore-forming species induced to sporulate. These are freeze dried into a powder form and subsequently blended with the spray-dried mix.

Apparatus suitable for processing of pollen as described above in Example 5 is shown in Figures 1 to 4, in which:

Figure 1 is a diagrammatic top view of drying apparatus;

Figure 2 is a fragmentary perspective view of one of the drying tubes of Figure 1 broken away to reveal an interior sleeve;

Figure 3 is a diagrammatic view of a shake table used to separate pollen grains from anthers; and

Figure 4 is a top plan view of the shake table of Figure 3.

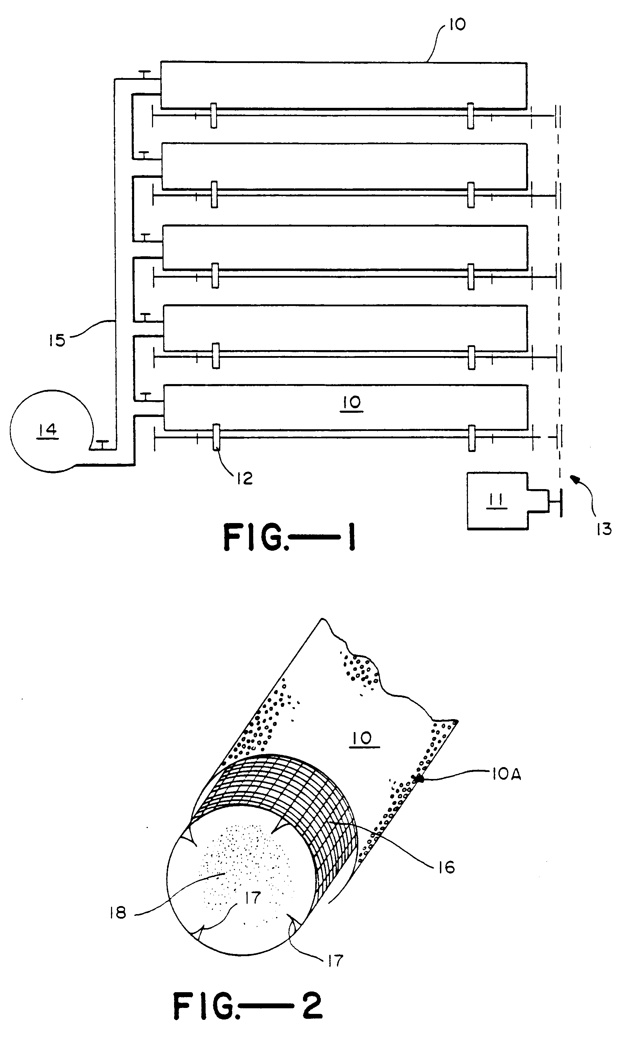

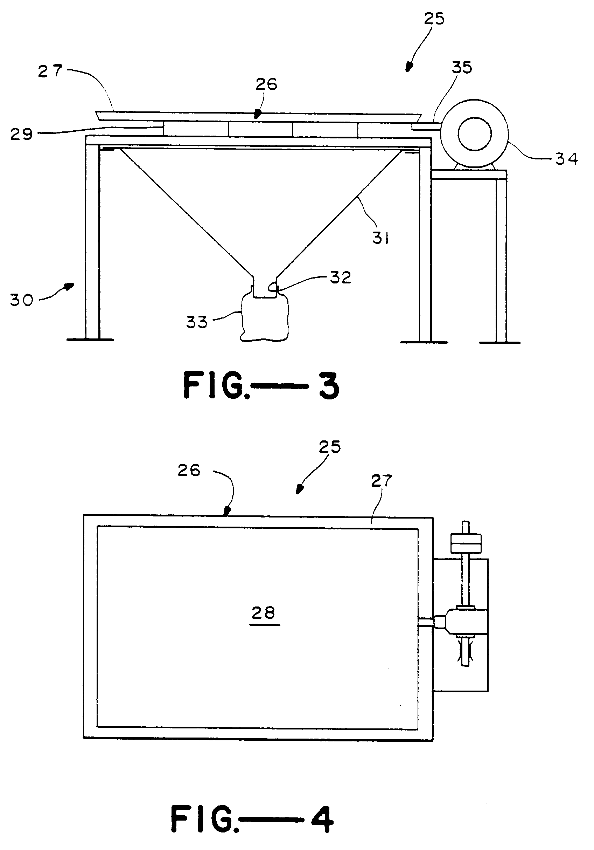

Referring now to Figures 1 and 2 a number of perforated cylinders 10, for example five in number, are provided which are suitably supported in horizontal position parallel to one another and are rotated about their longitudinal axes by a motor 11, rubber disks 12 bearing against the tubes and suitable connecting means indicated generally as 13 so that the tubes are rotated at a suitable speed, for example 15 to 30 rpm. An electric fan and heater 14 blows heated air through a manifold 15 and into the ends of the tubes 10. Preferably the air is maintained in a suitably dehumidified condition and at a suitable temperature, for example a moisture content of 20 to 40 relative humidity and a temperature of 18 to 25 degrees C. For example, the apparatus may be operated in a dehumidified room and the air is preferably treated chemically, for example by contact with potassium permanganate to eliminate potentially harmful substances such as ethylene and aromatics which are produced by organic material such as the anthers which are being treated, such material being harmful to the pollen.

Referring now to Figure 2, one of the cylinders 10 including its perforations 10A is shown and is broken away to reveal an inner sock or sleeve 16. The sock 16 is formed by stitching four segments of material together and is then turned inside out so that the unions 17 project inwardly to act as louvers to agitate and tumble the anthers which are shown at 18. The sock 16 is fixed to the interior surface of the cylinder 10 by any suitable means.

The sock 16 may be made of 62$\mu$m mesh (225 mesh) nylon, although other materials may be used and the mesh size will vary according to the species of anthers.

The duration of this drying will vary from case to case, a 24-hour period being typical. The dried anthers are then removed from the cylinders 10 and are placed on a shake table 25 which is shown in Figure 3. The drying process may be carried out continuously rather than batchwise.

Referring now to Figures 3 and 4, the shake table 25 comprises a tray 26 having a rim 27 and a perforated bottom 28 supported by flexible members 29 on a frame 30. A funnel 31 is supported by the frame 30 beneath the tray 26 and at its lower end the funnel is fitted with a spout 32 over which a bag 33 may be slipped.

A motor 34 is supported on the frame 30 and is connected by a reciprocating connector 35 to the tray 26. The bottom 28 of the tray is perforated, being conveniently formed by wire mesh screen having a mesh size such as to pass the liberated pollen grains but to hold back the remnants of the anthers left after crushing them to release the pollen grains. A suitable mesh size for anthers of almonds is about 90 $\mu$m mesh (170 mesh).

The motor 34 is operated to shake the tray at a suitable oscillatory speed, for example 400 to 500 cycles per minute. Meanwhile the anthers are gently rubbed by hand or by means of brushes, the pressure being sufficient to break open the anthers to liberate the pollen grains. The shaking action causes the pollen grains to fall through the screen 28 as they are released from the anthers, thereby limiting damage to the pollen grains due to the rubbing action.

The pollen may be processed and used as in Example 5 immediately or it may be stored, for example at 0 degrees C, for short periods of time or at-85 degrees C for long periods of time.

Further processing of the pollen is preferably carried out as described in Example 5.

The following is a list of crops to which the invention is applicable. The compositions applied are listed under Product and are applicable to each of the crops under a particular heading. Rates are gallons per acre or quarts per acre except in the case of the seed coating, Sun Coat.

Legend:

    a =    Bright Sun
    b =    Morning Sun
    c =    Super Sun Pollen
    d =    Asunder
    e =    Sun Burst
    f =    Sun Coat


**Crop**                          **Product**   **Rate**              **Applications**

**Cereal**:

**Rice**
   **(Uryza sativa)**
   **(Zizania aquatica)**

**Wheat**
   **(Triticum aestivum)**       a    $0.94\text{-}4.68 \text{ m}^1/\text{m}^2$ (1-5 gpa)          3-6

**Corn**
   **(Zea mays)**      .....     b    $0.24\text{-}0.94 \text{ m}^1/\text{m}^2$ (1-4 qt/a)          2-4

**Barley**
   **(Hordeum vulgare)**         f         -                  1

**Oats   (Avena sativa)**

**Sorghum   (Sorghum bicolor)**

**Rye   (secale cereale)**

**Millet   (various genera)**

Legumes

Soybean (Glycine max)

Peanut (Arachis hypogaea)

Beans (Phaseolus spp.)

Broad Bean
  (Pisum sativum)          a  $0.94\text{--}4.68\ \mathrm{m}^1/\mathrm{m}^2$ (1–5 gpa)     3–7

Pea
  (Pisum sativum) .....   b  $0.24\text{--}0.94\ \mathrm{m}^1/\mathrm{m}^2$ (1–4 qt/a)    2–4

Chickpea or Garbanzo      f            –                1
  (Cicer Arietinum)

Black Eyed Pea  (Vigna sinensis)

Lentil  (Lens spp.)

Pigeon Pea  (Cajanus indicus)

Guar  (Cyamopsis
    tetragonoloba)

**Forage Crops:**

**Alfalfa**  (Medicago sativa)

**Clover**
   (Trifolium spp.)          a    $0.94$–$4.68\ m^1/m^2$ (1–5 gpa)   **5–9**

**Bird's Foot Trefoil**       b    $0.24$–$0.94\ m^1/m^2$ (1–4 qt/a)   **2–4**
   (Lotus corniculatus)       c    $0.2$–$1\ mg/m^2$ (1–5 qt/a)    1–3

**Vetch**
   (Vicia spp.)              f          –                 **1**

**Sweet Clover**  (Meliolotus spp.)

**Lespedeza**  (Lespedeza spp.)

**Lupine**  (Lupinus spp.)

**Sorghum-Sudan**  (Sorghum spp.)

**Kentucky Bluegrass**        a    $0.94$–$2.8\ m^1/m^2$ (1–3 gpa)   **3–5**
   (Poa pratensis) .....      b    $0.24$–$0.94\ m^1/m^2$ (1–4 qt/a)   **2–4**

**Bromegrass**
   (Bromus spp.)             f                          **1**

**Timothy**  (Phleum pratense)

**Orchardgrass**  (Dactylis glomerata)

**Fescua**  (Festuca spp.)

**Bermudagrass**  (Cynodon spp.)

**Dallisgrass & Bahiagrass**
   (Paspalums spp.)

**Ryegrass**  (Lolium spp.)

**Bentgrass**  (Agrostis spp.)

<u>Stem and Leaf Crops</u>:

Sugar Cane  (Saccharum officinarum)

Artichoke  (Cynara scolymus)

Asparagus  (Asparagus officinalis)
(note:  repeated application
 in asparagus may allow more
 Spring cuttings)

Broccoli  (Brassica oleracea)

Brussels Sprouts
  (B. oleracea)                    **a**  0.94-4.68 m$^1$/m$^2$(1-5 gpa)    **4-7**

Cabbage
  (B. oleraces)    .....  **b** 0.24-0.94 m$^1$/m$^2$(1-4 qt/a)    **2-4**

Celery
  (Apium graveolens)        **f**

Chard  (Beta vulgaris)

Chinese Cabbage
  (Brassica campestris)

Collards  (B. oleracea)

Endive  (Cichorium endivia)

Kohlrabi  (B. oleracea)

Lettuce  (Lactuca sativa)

Parsley
  (Petroselinum sativum)

Rhubarb  (Rheum rhaponticum)

Spinach  (Spinacia oleracea)

<u>Root Crops</u>:

Potato   (Solanus tuberosum)

Cassave   (Manihot esculenta)

Sweet Potato   (Ipomoea batatas)

Beets   (Beta vulgaris)

Taro   (Colocasia spp.)

Carrot   (Daucus carota)

Horseradish                   **a**   $0.94\text{-}4.68\ m^1/m^2$(1-5 gpa)   **3-9**
   (Rorippa armoricia)

Jerusalem artichoke  ...  **b**   $0.24\text{-}0.94\ m^1/m^2$(1-4 qt/a)   **2-4**
   (Helianthus tuberosus) **f**                                        **1**

Onion   (Allium cepa)

Parsnip   (Pastinaca sativa)

Radish   (Raphanus sativus)

Rutabaga
   (Brassica napobrassica)

Salsify
   (Tragopogon porrifolius)

Turnip   (Brassica rapa)

Yam   (Diascorea spp.)


<u>Fruit and Seed Vegetables</u>:

Tomato                        **a**   $0.94\text{-}4.68\ m^1/m^2$(1-5 gpa)   **3-9**
   (Lycopersicon esculentum)

Eggplant
   (Solanum melongena) ..  **b**   $0.24\text{-}0.94\ m^1/m^2$ (1-4 qt/a)   **2-4**

Curcurbits                    **f**            -                **1**
   (various Curcurbitacea)

Okra   (Hibiscus esculentus)

Pepper   (Capsicum spp.)

Fruit and Nut Crops:

Citrus   (Citrus spp.)

Grape   (Vitis vinifera)

Banana   (Musa spp.)

Apple   (Malus spp.)

Stone Fruits   (Prunus spp.)

Blueberry (Vaccinium macrocarpon)

Brambles   (Rubus spp.)

Cranberry (Vaccinium macrocarpon)

Currant (Ribes sativum)

Pear      (Pyrus communis)

Avocado (Persea americana)

Cashew   (Anacardium occidentale)

Coconut
    (Cocos nucifera)        a  3.74–14.03 $m^1/m^2$ (4–15 gpa)  3–9

Date
    (Phoenix dactylifera)    b  0.24–0.94 $m^1/m^2$ (1–4 qt/a)  2–4

Fig
    (Ficus carica)           c  0.2–1 $mg/m^2$ (1–5 gr/a)    1–4

Guava
    (Psidium guajava)        d  3.74–14.03 $m^1/m^2$ (4–15 gpa)  1–2

Litchi
    (Litchi chinensis)       e  3.74–14.03 $m^1/m^2$ (4–15 gra)  2–3

Maracuja
    (Passiflora spp.)        f        as a root dip during
                                       planting

Mango     (Magnifera indica)

Olive     (Olea europea)

Papaya   (Carica papaya)

Pineapple (Ananas comosus)

Pomegranate   (Punica granatum)

Almond   (Prunus amygdalus)

Brazil Nut   (Bertholletia excelsa)

Filberts   (Corylus spp.)

Macadamia (Macadamis ternifolia)

Pecan (Carya illinoensis)

Pistachio (Pistacia vera)

Walnuts (Juglans spp.)

Sunflower (Helianthus annus)


Beverage Crops:

Coffee
   (Coffea arabica)          a   3.74–11.22 $m^1/m^2$(4–12 gpa)   3–9

Tea
   (Thea sinensis)           b   0.24–0.94 $m^1/m^2$(1–4 qt/a)   2–4

Cacao
   (Theobroma cacao)         f          as a root dip during
                                         planting

Cola     (Cola nitida)

Hops     (Humulus lupulus)


Oil, Fat and Wax Crops:

Safflower (Carthamus spp.)

Coconut   (Cocos nucifera)

African Oilpalm   (Elaeis Guineensis)

Castor Bean   (Ricinus commuis)

Rape
   (Brassica spp.)           a   0.94–4.68 $m^1/m^2$(1–5 gpa)   3–6

Sesame
   (Sesame indicum)          b   0.24–0.94 $m^1/m^2$ (1–4 qt/a)  2–4

Sunflower
   (Helianthus annus)        f          –              1
                                         also a root dip on
                                         selected crops

33

Linseed (linum usitatissimum)

Tung
  (Aleurites spp.)        d   $0.94\text{-}4.68\ m^1/m^2$(1-5 gpa)   1-3

Soybean (Glycine max)

Carnauba (Copernica cerifera)

Dandelilla   (Euphorbia antisyphilitica)

Jojoba   (Simmondsia chinensis)


<u>Spices, Perfumes and Flavorings</u>:

Black Pepper (Piper nigrum)

Cinnamon   (Cinnamomum zeylanicum)

Clove      (Eugenia caryophyllata)

Vanilla    (Vanilla planifolia)

Mint       (Mentha spp.)

Oregano    (Origanum spp.)

Allspice   (Pimenta officinalis)

Anise      (Pimpinella anisum)

Angelica Oil
  (Angelica spp.)         a   $0.94\text{-}4.68\ m^1/m^2$(1-5 gpa)   3-7

Mustard
  (Brassica spp.)         b   $0.24\text{-}0.94\ m^1/m^2$(1-4 qt/a)  2-4

Sage
  (Salvia officinalis)    f          -                1

Ginger   (Zingiber officinale)

Rose Oil   (Rosa spp.)

Bergamot   (Citrus aurantium bergamia)

Camphor   (Cinnamomum camphora)

Cananga   (Canangium odoratum)

Citronella Grass   (Cymbopogon nardus)

Eucalyptus (Eucalyptus citriodora)

EP 0 433 394 B1

```
Geranium Oil   (Perlargonium spp.)

Lavandula (Lavandula officinalis)

Rosemary   (Rosmarinus officinalis)

Thyme        (Thymus spp.)

Turpentine (Pinus spp.)


Ornamentals, Forest and Fiber Crops:

Cotton       (Gossypium spp.)

Flax         (Linum usitatissimum)

Hemp         (Canabis sativa)

Christmas Trees (various conifers)

Ornamental Evergreens     a   0.94–4.68 m$^{1}$/m$^{2}$(1–5 gpa)   3–10

Rose        (Rosa spp.)     b   0.24–0.94 m$^{1}$/m$^{2}$ (1–4 qt/a)   2–4

Chrysanthemum               f                               1
     (Chrysanthemum spp.)

Carnation (Dianthus spp.)
(or as root dip)

Iris         (Iris spp.)

Azalea and Rhododendron
     (Azalea spp.)

Houseplants (various species)
```

It will therefore be apparent that a novel composition of matter for and a novel method of treating a variety of plants to improve such things as growth, crop yield, resistance to pests and resistance to frost have been provided.

**Claims**

1. A composition of matter useful as a foliar spray to stimulate growth of plants, such composition comprising the following components substantially completely dissolved in an aqueous medium:
   (a) a carbon skeleton/energy component
   (b) a macronutrient component
   (c) a micronutrient component
   the carbon skeleton/energy component (a) being one or more organic compounds which are water soluble and which are assimilable by plants to provide energy required by metabolism of the plant and to provide carbon skeleton precursors for synthesis of proteins and other plant components,
   the macronutrient component (b) comprising water-soluble assimilable compounds of the elements nitrogen, phosphorus, potassium and calcium,
   the micronutrient component (c) comprising water-soluble assimilable compounds of the elements zinc, iron and manganese,
   component (a) being present in an amount to provide 4 to 10% of total invert sugar,
   each of said components (b) and (c) being present in quantity smaller than component (a) but sufficient to perform its intended function when applied to a plant by foliar spraying,
   the composition also including

(d) a vitamin/cofactor component selected and in a quantity to stimulate energy production and biosynthesis by the plant such that the burden of energy production and carbon skeleton production by component (a) is shared by photosynthesis and other biosynthetic paths of the plant, the amount of component (d) and of its sub-components being smaller than component (a) but consistent with metabolism and biosynthesis of the plant.

2.  The composition of Claim 1 including also an enhancement component (e), such component acting to facilitate transfer of other components into the cell structure of the plant.

3.  The composition of Claim 1 wherein component (a) is a sugar or mixture of sugars.

4.  The composition of Claim 3 wherein the sugars are in the form of molasses.

5.  The composition of Claim 1 wherein component (b) includes water soluble, assimilable magnesium and sulfur and component (c) includes water-soluble assimilable copper, boron, molybdenum and cobalt.

6.  A method of treating a plant to stimulate its growth and/or its maturation or the production of a crop, such method comprising applying to the plant an aqueous solution of the following components substantially completely dissolved in such aqueous solution:

    (a) a carbon skeleton/energy component
    (b) a macronutrient component
    (c) a micronutrient component

    the carbon skeleton/energy component (a) being one or more organic compounds which are water soluble and which are assimilable by plants to provide energy required by metabolism of the plant and to provide carbon skeleton precursors for synthesis of proteins and other plant components,

    the macronutrient component (b) comprising water-soluble assimilable compounds of the elements nitrogen, phosphorus, potassium and calcium,

    the micronutrient component (c) comprising water-soluble assimilable compounds of the elements zinc, iron and manganese,

    component (a) being present in an amount to provide 4 to 10% of total invert sugar,

    each of said components (b) and (c) being present in quantity smaller than component (a) but sufficient to perform its intended function when applied to a plant by foliar spraying,

    the solution also including

    (d) a vitamin/cofactor component selected and in a quantity to stimulate energy production and biosynthesis by the plant such that the burden of energy production and carbon skeleton production by component (a) is shared by photosynthesis and other biosynthetic paths of the plant, the amount of component (d) and of its sub-components being smaller than component (a) but consistent with metabolism and biosynthesis of the plant.

7.  The method of Claim 6 wherein the solution is applied as a spray to the foliage of the plant during the early spring flush through the mid-summer growing season, such application being in increments spaced apart timewise.

8.  The method of Claim 7 wherein the plant is an annual plant or is a perennial plant which produces an edible crop and the application of the solution is carried out at intervals including one or more applications during the prelog phase and one or more applications during log phase of growth of the plant or of its crop and one or more applications during the linear phase of growth of the plant or its crop, the total applications being substantially less than that theoretically required by the plant for growth during such period of time, the frequency and spacing of applications and the rate of each application being carried out consistently with energy/carbon skeleton, macro- and micro-nutrient requirements at each stage of growth of the plant.

9.  The method of Claim 8 wherein said composition includes also an enhancement component (e), such component acting to facilitate transfer of other components into the cell structure of the plant.

10. The method of Claim 9 wherein the component (a) is a sugar or mixture of sugars.

11. The method of Claim 10 wherein the sugars are in the form of molasses.

**12.** The method of Claim 6 wherein the component (b) includes water soluble, assimilable magnesium and sulfur and component (c) includes water-soluble, assimilable copper, boron, molybdenum and cobalt.

**13.** The method of Claim 6 wherein the component (a) is an amino acid or derivative thereof.

**14.** The method of Claim 6 wherein the component (a) is a sugar alcohol.

**15.** The method of Claim 7 wherein the plants which are treated are almond trees.

**16.** The method of Claim 7 wherein the plants which are treated are pistachio trees.

**17.** The method of Claim 6 wherein pollen separated from anthers is treated with the composition of Claim 1 and the treated pollen is used to pollinate blossoms.

**Patentansprüche**

**1.** Gemisch für die Verwendung als Blattspray für die Stimulierung des Pflanzenwachstums, das die nachfolgend aufgeführten Komponenten in einem wässerigen Medium im wesentlichen vollständig gelöst enthält:
(a) eine das Kohlenstoffgerüst bildende und Energie liefernde Komponente,
(b) eine Makronährstoffkomponente und
(c) eine Mikronährstoffkomponente,
wobei die das Kohlenstoffgerüst bildende und Energie liefernde Komponente (a) eine oder mehrere organische Verbindungen darstellt, die wasserlöslich sind und von den Pflanzen assimiliert werden können, um die für den Stoffwechsel der Pflanze benötigte Energie zu liefern und die Precursorverbindungen des Kohlenstoffgerüstes für die Proteinsynthese und andere Komponenten der Pflanzen bereitzustellen,
die Makronährstoffkomponente (b) wasserlösliche, assimilierbare Verbindungen der Elemente Stickstoff, Phosphor, Kalium und Calcium umfaßt,
die Mikronährstoffkomponente (c) wasserlösliche, assimilierbare Verbindungen der Elemente Zink, Eisen und Mangan umfaßt,
die Komponente (a) in einer Menge von 4 bis 10%, bezogen auf den Gesamtinvertzucker, vorliegt,
jede der Komponenten (b) und (c) in einer geringeren Menge als die Komponente (a) vorliegt, wobei diese Menge jedoch ausreicht, um beim Aufbringen auf eine Pflanze durch Besprühen der Blätter die beabsichtigte Wirkung zu erzielen,
das Gemisch außerdem noch
(d) eine Vitamin- bzw. Cofaktorkomponente umfaßt, die so ausgewählt wird und in einer solchen Menge, daß die Energiegewinnung und Biosynthese in der Pflanze angeregt werden, so daß sich die Last der Energiegewinnung und Kohlenstoffgerüstbildung durch die Komponente (a) auf Photosynthese und andere Biosynthesewege der Pflanze verteilt, die Menge der Komponente (d) und ihrer Unterkomponenten geringer ist als die Komponente (a), jedoch mit dem Stoffwechsel und der Biosynthese der Pflanze im Einklang steht.

**2.** Gemisch nach Anspruch 1, das außerdem noch eine Verstärkungskomponente (e) umfaßt, welche den Transport anderer Komponenten in die Zellen der Pflanzen erleichtert.

**3.** Gemisch nach Anspruch 1, bei dem die Komponente (a) ein Zucker oder ein Zuckergemisch ist.

**4.** Gemisch nach Anspruch 3, bei dem die Zucker in Form von Molasse vorliegen.

**5.** Gemisch nach Anspruch 1, bei dem die Komponente (b) wasserlösliches, assimilierbares Magnesium und wasserlöslichen, assimilierbaren Schwefel und die Komponente (c) wasserlösliches, assimilierbares Kupfer, Bor, Molybdän und Kobalt umfaßt.

**6.** Verfahren zur Behandlung von Pflanzen zur Stimulierung ihres Wachstums und/oder ihrer Reifung bzw. der Produktion von Früchten, wobei bei diesem Verfahren auf die Pflanze eine wässerige Lösung der nachfolgend genannten, im wesentlichen in der wässerigen Lösung vollständig gelösten Komponenten aufgebracht wird:

(a) eine das Kohlenstoffgerüst bildende und Energie liefernde Komponente

(b) eine Makronährstoffkomponente und

(c) eine Mikronährstoffkomponente,

wobei die das Kohlenstoffgerüst bildende und Energie liefernde Komponente (a) eine oder mehrere organische Verbindungen darstellt, die wasserlöslich sind und von den Pflanzen assimiliert werden können, um die für den Stoffwechsel der Pflanze benötigte Energie zu liefern und die Precursorverbindungen des Kohlenstoffgerüstes für die Proteinsynthese und andere Komponenten der Pflanzen bereitzustellen,

die Makronährstoffkomponente (b) wasserlösliche, assimilierbare Verbindungen der Elemente Stickstoff, Phosphor, Kalium und Calcium umfaßt,

Die Mikronährstoffkomponente (c) wasserlösliche, assimilierbare Verbindungen der Elemente Zink, Eisen und Mangan umfaßt,

die Komponente (a) in einer Menge von 4 bis 10%, bezogen auf den Gesamtinvertzucker vorliegt,

jede der Komponenten (b) und (c) in einer geringeren Menge als die Komponente (a) vorliegt, wobei diese Menge jedoch ausreicht, um beim Aufbringen auf eine Pflanze durch Besprühen der Blätter die beabsichtigte Wirkung zu erzielen,

die Lösung außerdem noch

(d) eine Vitamin- bzw. Cofaktorkomponente umfaßt, die so ausgewählt wird und in einer solchen Menge, daß die Engeriegewinnung und Biosynthese in der Pflanze angeregt werden, so daß sich die Last der Energiegewinnung und Kohlenstoffgerüstbildung durch die Komponente (a) auf Photosynthese und andere Biosynthesewege der Pflanze verteilt, die Menge der Komponente (d) und ihrer Unterkomponenten geringer ist als die Komponente (a), jedoch mit dem Stoffwechsel und der Biosynthese der Pflanze im Einklang steht.

7. Verfahren nach Anspruch 6, worin die Lösung als Spray auf die Blätter der Pflanze während des Austreibens im Vorfrühling bis zur Vegetationsperiode im Hochsommer aufgebracht wird, und zwar in zeitlichen Abständen bei zunehmenden Mengen.

8. Verfahren nach Anspruch 7, worin die Pflanze eine einjährige oder mehrjährige Pflanze ist, die eine eßbare Frucht hervorbringt und die Aufbringung der Lösung in Intervallen mit einem oder mehreren Aufbringungsschritten während der prelog-Phase und einer oder mehreren Aufbringungen während der log-Phase des Wachstums der Pflanze bzw. ihrer Frucht und einer oder mehreren Aufbringungen während der linearen Phase des Wachstums der Pflanze bzw. ihrer Frucht erfolgt, wobei die Gesamtheit der Aufbringungsschritte im wesentlichen unter der für das Pflanzenwachstum während dieses Zeitabschnitts theoretisch erforderlichen Aufbringungen liegt, die Frequenz und der Zeitraum zwischen den einzelnen Aufbringungen und die Aufbringungsgeschwindigkeit jeweils entsprechend den Erfordernissen im Hinblick auf Energiegewinnung und Bildung des Kohlenstoffgerüsts sowie Makro- und Mikronährstoffe auf jeder Stufe des Pflanzenwachstums festgelegt werden.

9. Verfahren nach Anspruch 8, bei dem das Gemisch außerdem noch eine Verstärkungskomponente (e) umfaßt, welche den Transport anderer Komponenten in die Zellen der Pflanzen erleichtert.

10. Verfahren nach Anspruch 9, bei dem die Komponente (a) ein Zucker oder ein Zuckergemisch ist.

11. Verfahren nach Anspruch 10, bei dem die Zucker in Form von Molasse vorliegen.

12. Verfahren nach Anspruch 6, bei dem die Komponente (b) wasserlösliches, assimilierbares Magnesium und wasserlöslichen, assimilierbaren Schwefel und die Komponente (c) wasserlösliches, assimilierbares Kupfer, Bor, Molybdän und Kobalt umfaßt.

13. Verfahren nach Anspruch 6, bei dem die Komponente (a) eine Aminosäure oder ein Derivat davon ist.

14. Verfahren nach Anspruch 6, bei dem die Komponente (a) ein Zuckeralkohol ist.

15. Verfahren nach Anspruch 7, bei dem die Pflanzen, die behandelt werden, Mandelbäume sind.

16. Verfahren nach Anspruch 7, bei dem die Pflanzen, die behandelt werden, Pistazienbäume sind.

EP 0 433 394 B1

**17.** Verfahren nach Anspruch 6, bei dem der von den Antheren abgetrennte Pollen mit dem Gemisch nach Anspruch 1 behandelt wird und der behandelte Pollen zur Bestäubung von Blüten verwendet wird.

**Revendications**

**1.** Composition utile en pulvérisation foliaire pour stimuler la croissance de plantes, ladite composition comprenant les constituants suivants dissous pratiquement totalement dans un milieu aqueux :

(a) un constituant fournissant un squelette carboné/de l'énergie

(b) un constituant macronutritif

(c) un constituant micronutritif

le constituant fournissant un squelette carboné/de l'énergie (a) consistant en un ou plusieurs composés organiques qui sont hydrosolubles et qui sont assimilables par des plantes pour fournir l'énergie requise par le métabolisme de la plante et pour fournir des précurseurs de squelette carboné pour la synthèse de protéines et d'autres constituants de la plante,

le constituant macronutritif (b) comprenant des composés, hydrosolubles assimilables, des éléments consistant en azote, phosphore, potassium et calcium,

le constituant micronutritif (c) comprenant des composés, hydrosolubles assimilables, des éléments consistant en zinc, fer et manganèse,

le constituant (a) étant présent en une quantité destinée à fournir 4 à 10 % de sucres inversés totaux,

chacun desdits constituants (b) et (c) étant présent en une quantité inférieure à celle du constituant (a) mais suffisante pour jouer son rôle envisagé lors de l'application à une plante par pulvérisation foliaire,

la composition comprenant également

(d) un constituant vitaminique/à action de cofacteur choisi et en une quantité pour stimuler la production d'énergie et la biosynthèse par la plante de telle sorte que la charge de production d'énergie et de production de squelette carboné par le constituant (a) soit partagée par la photosynthèse et les autres voies biosynthétiques de la plante, la quantité de constituant (d) et de ses constituants secondaires étant inférieure à celle du constituant (a) mais en accord avec le métabolisme et la biosynthèse de la plante.

**2.** Composition suivant la revendication 1, comprenant également un constituant activateur (e), ledit constituant ayant pour rôle de faciliter le transfert d'autres constituants dans la structure cellulaire de la plante.

**3.** Composition suivant la revendication 1, dans laquelle le constituant (a) est un sucre ou mélange de sucres.

**4.** Composition suivant la revendication 3, dans laquelle les sucres sont sous forme de mélasse.

**5.** Composition suivant la revendication 1, dans laquelle le constituant (b) comprend du magnésium et du soufre hydrosolubles assimilables, et le constituant (c) comprend du cuivre, du bore, du molybdène et du cobalt hydrosolubles assimilables.

**6.** Procédé de traitement d'une plante pour stimuler sa croissance et/ou sa maturation ou la production d'une culture, ledit procédé comprenant l'application à la plante d'une solution aqueuse des constituants suivants dissous pratiquement totalement dans ladite solution aqueuse :

(a) un constituant fournissant un squelette carboné/de l'énergie

(b) un constituant macronutritif

(c) un constituant micronutritif

le constituant fournissant un squelette carboné/de l'énergie (a) consistant en un ou plusieurs composés organiques qui sont hydrosolubles et qui sont assimilables par des plantes pour fournir l'énergie requise par le métabolisme de la plante et pour fournir des précurseurs de squelette carboné pour la synthèse de protéines et d'autres constituants de la plante,

le constituant macronutritif (b) comprenant des composés hydrosolubles assimilables des éléments consistant en azote, phosphore, potassium et calcium,

le constituant micronutritif (c) comprenant des composés hydrosolubles assimilables des éléments consistant en zinc, fer et manganèse,

39

le constituant (a) étant présent en une quantité choisie de manière à fournir 4 à 10 % de sucres inversés totaux,

chacun desdits constituants (b) et (c) étant présent en une quantité inférieure à celle du constituant (a) mais suffisante pour qu'il joue son rôle envisagé lors de l'application à une plante par pulvérisation foliaire,

la solution comprenant également

(d) un constituant vitaminique/à effet de cofacteur choisi et en une quantité pour stimuler la production d'énergie et la biosynthèse par la plante de telle sorte que la charge de production d'énergie et de production de squelette carboné par le constituant (a) soit partagée par la photosynthèse et les autres voies biosynthétiques de la plante, la quantité de constituant (d) et de ses constituants secondaires étant inférieure à celle du constituant (a) mais en accord avec le métabolisme et la biosynthèse de la plante.

7. Procédé suivant la revendication 6, dans lequel la solution est appliquée par pulvérisation au feuillage de la plante au cours de la pousse de premier printemps jusqu'à la période de croissance de milieu d'été, cette application étant effectuée par portions successives espacées dans le temps.

8. Procédé suivant la revendication 7, dans lequel la plante est une plante annuelle ou est une plante pérenne qui donne une culture comestible et l'application de la solution est effectuée par intervalles, comprenant une ou plusieurs applications au cours de la phase prélogarithmique et une ou plusieurs applications au cours de la phase logarithmique de croissance de la plante ou de la culture obtenue avec cette plante, et une ou plusieurs applications au cours de la phase linéaire de croissance de la plante ou de la culture obtenue avec cette plante, les applications totales étant notablement inférieures aux applications théoriquement requises par la plante pour la croissance au cours de cette période de temps, la fréquence et l'espacement des applications et la vitesse de chaque application étant en accord avec les besoins en énergie/squelette carboné et substances macro- et micronutritives à chaque stade de croissance de la plante.

9. Procédé suivant la revendication 8, dans lequel la composition comprend également un constituant activateur (e), ce constituant ayant pour rôle de faciliter le transfert d'autres constituants dans la structure cellulaire de la plante.

10. Procédé suivant la revendication 9, dans lequel le constituant (a) est un sucre ou mélange de sucres.

11. Procédé suivant la revendication 10, dans lequel les sucres sont sous forme de mélasse.

12. Procédé suivant la revendication 6, dans lequel le constituant (b) comprend du magnésium et du soufre hydrosolubles assimilables, et le constituant (c) comprend du cuivre, du bore, du molybdène et du cobalt hydrosolubles assimilables.

13. Procédé suivant la revendication 6, dans lequel le constituant (a) est un amino-acide ou un de ses dérivés.

14. Procédé suivant la revendication 6, dans lequel le constituant (a) est un sucre-alcool.

15. Procédé suivant la revendication 7, dans lequel les plantes qui sont traitées sont des amandiers.

16. Procédé suivant la revendication 7, dans lequel les plantes qui sont traitées sont des pistachiers.

17. Procédé suivant la revendication 6, dans lequel le pollen séparé des anthères est traité avec la composition suivant la revendication 1 et le pollen traité est utilisé pour polliniser des fleurs.

FIG.—1

FIG.—2

FIG.— 3

FIG.— 4